# EUROPÄISCHE PATENTSCHRIFT

(11) **EP 2 571 873 B1**
(45) Veröffentlichungstag und Bekanntmachung des Hinweises auf die Patenterteilung: **24.06.2015**
(21) Anmeldenummer: 11721487.4
(22) Anmeldetag: 17.05.2011
(51) Int. Cl.: C07D 417/14, A01N 43/78, C07D 471/14

(54) **BIS(DIFLUORMETHYL)PYRAZOLE ALS FUNGIZIDE**
BIS(DIFLUORMETHYL)PYRAZOLES AS FUNGICIDES
BIS(DIFLUORMÉTHYL)PYRAZOLES EN TANT QUE FONGICIDE

(30) Priorität: 18.05.2010 US 345796 P; 18.05.2010 EP 10163067
(43) Veröffentlichungstag der Anmeldung: 27.03.2013
(73) Patentinhaber: Bayer CropScience AG, 40789 Monheim (DE)
(72) Erfinder: CRISTAU, Pierre, F-69009 Lyon (FR); HOFFMANN, Sebastian, 41470 Neuss (DE); KLUTH, Joachim, 40764 Langenfeld (DE); SEITZ, Thomas, 40764 Langenfeld (DE); TSUCHIYA, Tomoki, 69009 Lyon (FR); WASNAIRE, Pierre, 40225 Düsseldorf (DE); BENTING, Jürgen, 42799 Leichlingen (DE); WACHENDORFF-NEUMANN, Ulrike, 56566 Neuwied (DE)
(74) Vertreter: BIP Patents
(86) Internationale Anmeldenummer: PCT/EP2011/057912
(87) Internationale Veröffentlichungsnummer: WO 2011/144586

(56) Entgegenhaltungen:
- WO-A2-2007/014290

## Beschreibung

Die vorliegende Erfindung betrifft neue Bis(difluormethyl)pyrazol-Derivate, Verfahren zu deren Herstellung, deren Verwendung zum Bekämpfen von unerwünschten Mikroorganismen, insbesondere phytopathogener Pilze, im Pflanzenschutz, im Bereich Haushalt und Hygiene und im Materialschutz, sowie Pflanzenschutzmittel enthaltend diese Bis(difluormethyl)pyrazol-Derivate.

Es ist bereits bekannt, dass bestimmte substituierte Pyrazol-Derivate als fungizide Pflanzenschutzmittel benutzt werden können (siehe WO 2007/014290, WO 2008/013925, WO 2008/013622, WO 2008/091594, WO 2008/091580, WO 2009/055514, WO 2009/094407, WO 2009/094445). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Bis(difluormethyl)pyrazol-Derivate die genannten Aufgaben zumindest in Teilaspekten lösen und sich als Pflanzenschutzmittel, insbesondere als Fungizide eignen.

Gegenstand der Erfindung sind Verbindungen der Formel **(I)**, 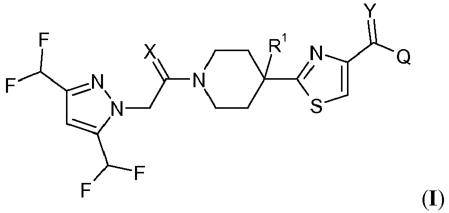 in welcher die Symbole folgende Bedeutungen haben:
- X: steht für Sauerstoff oder Schwefel,
- R¹: steht für Wasserstoff oder Halogen,
- Y: steht für Sauerstoff oder Schwefel,
- Q: steht für -NR²R³,
- R²: steht für Wasserstoff, C₁-C₃-Alkyl, C₂-C₃-Alkenyl, C₂-C₃-Alkinyl, C₄-C₁₀-Cycloalkylalkyl, C₄-C₁₀-Alkylcycloalkyl, C₅-C₁₀-Alkylcycloalkylalkyl, C₁-C₃-Halogenalkyl, C₂-C₃-Halogenalkenyl, Cyano, Hydroxy, C₁-C₃-Alkoxy, C₂-C₃-Alkoxyalkyl, C₁-C₃-Hydroxyalkyl, C₂-C₃-Alkylcarbonyl, C₂-C₃-Alkoxycarbonyl, C₂-C₃-Alkylaminocarbonyl oder C₃-C₅-Dialkylaminocarbonyl,
- R³: steht für ein 8- bis 11-gliedriges, unsubstituiertes oder substituiertes, gesättigtes oder teilweise gesättigtes bicyclisches Ringsystem oder steht für ein 10- bis 15-gliedriges, unsubstituiertes oder substituiertes, gesättigtes oder teilweise gesättigtes tricyclisches Ringsystem; jedes Ringsystem kann gegebenenfalls ein bis drei Heteroatome enthalten (bis zu ein Sauerstoffatom, bis zu ein Schwefelatom und bis zu drei Stickstoffatomen) und gegebenenfalls ein bis drei Ringglieder, die aus der Gruppe C(=O), C(=S), S(=O) oder S(=O)₂ ausgewählt werden können
oder
R² und R³ bilden zusammen mit dem Stickstoffatom, an das sie gebunden sind, einen 5- oder 6-gliedrigen, unsubstituierten oder substituierten, gesättigten oder teilweise gesättigten Heterocyclylrest
oder
- R³: steht für -CR⁴R⁵R⁶,
- R⁴: steht für Wasserstoff, C₁-C₆-Alkyl, C₂-C₆-Alkenyl, C₂-C₆-Alkinyl, C₃-C₆-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, Cyano, Nitro, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylaminocarbonyl, C₃-C₈-Dialkylaminocarbonyl oder C₃-C₆-Trialkylsilyl,
- R⁵: steht für unsubstituierts oder substituierts Phenyl, Benzyl, Naphthalenyl, C₃-C₆-Cycloalkyl, C₃-C₆-Cycloalkenyl oder einen 5- oder 6-gliedrigen Heteroarylrest,
- R⁶: steht für Wasserstoff, C₁-C₄-Alkyl, C₂-C₄-Alkenyl, C₂-C₄-Alkynyl, C₃-C₄-Cycloalkyl, C₄-C₁₀-Cycloalkylalkyl, C₄-C₁₀-Alkylcycloalkyl, C₅-C₁₀-Alkylcycloalkylalkyl, C₁-C₄-Halogenalkyl, C₂-C₄-Halogenalkenyl, C₂-C₄-Halogenalkinyl, C₃-C₄-Halogencycloalkyl oder C₂-C₄-Alkoxyalkyl,
oder
- R² und R⁴: bilden zusammen mit den Atomen, an die sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu zwei Stickstoffatomen),
oder
- R² und R⁵: bilden zusammen mit den Atomen, an die sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu zwei Stickstoffatomen),
oder
- R⁴ und R⁶: bilden zusammen mit dem Kohlenstoff, an den sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu ein Stickstoffatom),
oder
- R⁴ und R⁵: bilden zusammen mit dem Kohlenstoff, an den sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu ein Stickstoffatom),
sowie agrochemisch wirksame Salze davon.

Ein weiterer Gegenstand der Erfindung ist die Verwendung der Verbindungen der Formel (I) als Fungizid.

Erfindungsgemäße Bis(difluormethyl)pyrazol-Derivate der Formel (**I**) sowie deren agrochemisch wirksame Salze eignen sich sehr gut zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (**I**) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäß verwendbaren Bis(difluormethyl)pyrazol-Derivate sind durch die Formel (**I**) allgemein definiert. Bevorzugte Restedefinitionen der vorstehenden und nachfolgend genannten Formeln sind im Folgenden angegeben. Diese Definitionen gelten für die Endprodukte der Formel (**I**) wie für alle Zwischenprodukte (siehe auch unten unter "Erläuterungen der Verfahren und Zwischenprodukte) gleichermaßen.
- X: steht bevorzugt für Sauerstoff,
- R¹: steht bevorzugt für Wasserstoff oder Fluor,
- Y: steht bevorzugt für Sauerstoff,
- Q: steht bevorzugt für, 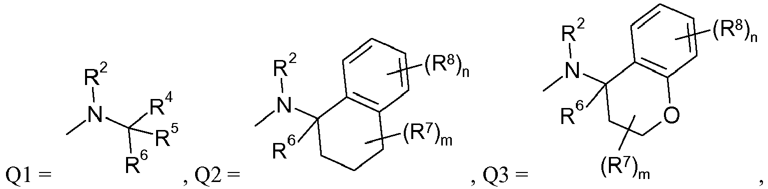 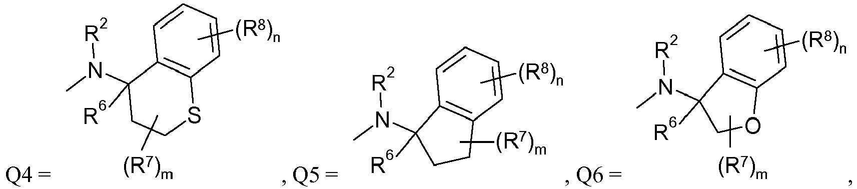 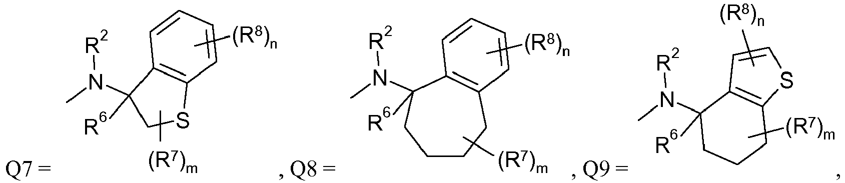 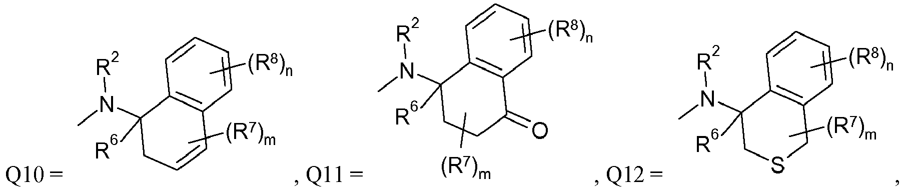 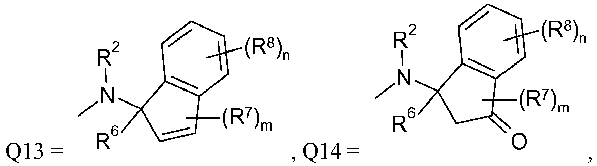 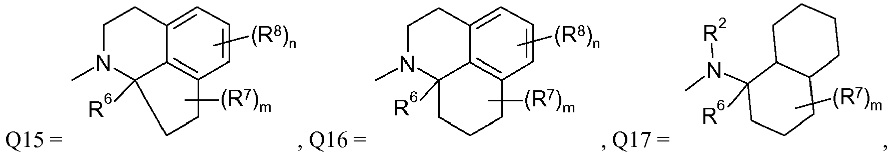 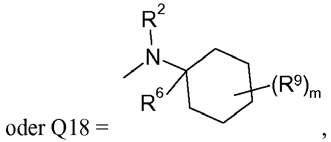
- Q: steht besonders bevorzugt für Q1, Q2, Q5, Q9, Q11, Q14, Q15 oder Q16,
- Q: steht ganz besonders bevorzugt für Q2, Q5 oder Q9,
- Q: steht insbesondere bevorzugt für Q2,
- m: steht bevorzugt für 0, 1 oder 2,
- m: steht besonders bevorzugt für 0 oder 1,
- m: steht ganz besonders bevorzugt für 0,
- n: steht bevorzugt für 0, 1 oder 2,
- n: steht besonders bevorzugt für 0 oder 1,
- n: steht ganz besonders bevorzugt für 0,
- R²: steht bevorzugt für Wasserstoff oder C₁-C₃-Alkyl,
- R²: steht besonders bevorzugt für Wasserstoff, Methyl, Ethyl oder Isopropyl,
- R²: steht ganz besonders bevorzugt für Wasserstoff oder Methyl,
- R⁴: steht bevorzugt für Wasserstoff, C₁-C₄-Alkyl, C₃-C₆-Cycloalkyl, C₂-C₄-Alkoxyalkyl, C₁-C₆-Halogenalkyl,
- R⁴: steht besonders bevorzugt für Wasserstoff oder C₁-C₄-Alkyl,
- R⁴: steht ganz besonders bevorzugt für Wasserstoff, Methyl oder Ethyl,
- R⁵: steht bevorzugt für unsubstituiertes oder substituiertes Phenyl, Benzyl oder C₃-C₆-Cycloalkyl,
- R⁵: steht besonders bevorzugt für Phenyl, das bis zu drei Substituenten enthalten kann, wobei die Substituenten unabhängig voneinander aus folgender Liste ausgewählt sind: Halogen, Cyano, Nitro, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalky C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl oder Tri(C₁-C₄-alkyl)silyl,
- R⁵: steht ganz besonders bevorzugt für Phenyl, das bis zu drei Substituenten enthalten kann, wobei die Substituenten unabhängig voneinander aus folgender Liste ausgewählt sind: Chlor, Fluor, Brom, Iod, Cyano, Nitro, -CH₃, -CH₂CH₃, -CH₂CH₂CH₃, -CH(CH₃)₂, -CH₂CH₂CH₂CH₃,-CH(CH₃)CH₂CH₃, -CH₂CH(CH₃)CH₃, -C(CH₃)₃, -CH=CH₂, -CH=CHCH₃, -CH₂CH=CH₂,-CH=CHCH₂CH₃, -CH₂CH=CHCH₃, -CH₂CH₂CH=CH₂, -C≡CH, -C≡CCH₃, -CH₂C≡CH,-C≡CCH₂CH₃, -CH₂C≡CCH₃, -CH₂CH₂C≡CH, -CF₃, -CFH₂, -CF₂H, -CF₂CF₃, -CCl₃, Cyclopropyl, Cyclobutyl, Cyclopentyl, Cyclohexyl, -CH₂OCH₃, -CH₂OCH₂CH₃, -CH₂CH₂OCH₃, -CH₂OCH₂CH₂CH₃, -CH₂CH₂OCH₂CH₃, -CH₂CH₂CH₂OCH₃, -C(=O)CH₃, -C(=O)CH₂CH₃, C(=O)CH₂CH₂CH₃, C(=O)CH(CH₃)₂, -C(=O)CF₃, -C(=O)OCH₃, -C(=O)OCH₂CH₃, - C(=O)OCH₂CH₂CH₃, -C(=O)OCH(CH₃)₂, -OCH₃, -OCH₂CH₃, -OCH₂CH₂CH₃, - OCH(CH₃)₂, -OCH₂CH₂CH₂CH₃, -OCH₂CH(CH₃)₂, -OCH(CH₃)CH₂CH₃, -OC(CH₃)₃, - OCF₃, -OCF₂H, -OCH₂CF₃, -OCF₂CF₃. O-Cyclohexyl, O-Cyclopentyl, O-Cyclopropyl, - SCH₃, -SCH₂CH₃, -SCH₂CH₂CH₃, -SCH(CH₃)₂, -SCH₂CH₂CH₂CH₃, -SCH₂CH(CH₃)₂, - SCH(CH₃)CH₂CH₃, -SC(CH₃)₃, -SCF₃, -SCF₂H, -SCH₂CF₃, -SCF₂CF₃, -S(=O)Me,-S(O)CF₃, -S(=O)₂Me, -S(O)₂CF₃, -OCH₂CH=CH₂, -OCH₂C≡CH, -OCH₂OCH₃, - OCH₂OCH₂CH₃, -OCH₂CH₂OCH₃, -OCH₂OCH(CH₃)₂ oder Trimethylsilyl,
- R⁶: steht bevorzugt für Wasserstoff oder C₁-C₄-Alkyl, C₃-C₄-Cycloalkyl, C₂-C₄-Alkoxyalkyl, C₁-C₄-Halogenalkyl,
- R⁶: steht besonders bevorzugt für Wasserstoff oder C₁-C₄-Alkyl,
- R⁶: steht ganz besonders bevorzugt für Wasserstoff, Methyl oder Ethyl
- R⁷: steht jeweils unabhängig voneinander bevorzugt für Wasserstoff, C₁-C₄-Alkyl, Halogen, Cyano, Hydroxy, Amino, Nitro, C₂-C₄-Alkenyl, C₃-C₄-Alkinyl, C₁-C₄-Halogenalkyl, C₂-C₄-Halogenalkenyl, C₂-C₄-Halogenalkinyl, C₃-C₆-Cycloakl, C₃-C₆-Halogencycloakyl, C₂-C₄-Alkoxyalkyl, C₁-C₄-Alkylamino, C₂-C₆-Dialkylamino, C₃-C₆-Cycloaklamino, C₂-C₄-Alkylcarbonyl, C₂-C₄-Alkoxycarbonyl, C₂-C₄-Alkylaminocarbonyl, C₂-C₄-Alkylaminocarbonyloxy, C₃-C₆-Dialkylaminocarbonyl, C₁-C₄-Hydroxyalkyl, C₁-C₄-Alkoxy, C₁-C₄-Halogenalkoxy, C₂-C₄-Alkylcarbonyloxy, C₂-C₄-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Tri(C₁-C₂-alkyl)silyl,
- R⁷: steht jeweils unabhängig voneinander besonders bevorzugt für Wasserstoff, C₁-C₄-Alkyl, Halogen, Cyano, Hydroxy, Amino, Nitro, C₂-C₄-Alkenyl, C₃-C₄-Alkinyl, C₁-C₄-Halogenalkyl, C₂-C₄-Halogenalkenyl, C₂-C₄-Halogenalkinyl, C₃-C₆-Cycloalkyl, C₃-C₆-Halogencycloalkyl, C₁-C₄-Alkoxy oder C₂-C₄-Alkylcarbonyloxy,
- R⁷: steht jeweils unabhängig voneinander ganz besonders bevorzugt für Wasserstoff, Methyl, Ethyl, -CF₃, -CFH₂, -CF₂H, -CF₂CF₃, -CCl₃, -OCH₃, -OCH₂CH₃, -OCF₃, -OCF₂H, -OCH₂CF₃, -OCF₂CF_{3.} -OC(=O)CH₃, -OC(=O)CH₂CH₃, -OC(=O)CH₂CH₂CH₃, -OC(=O)CH(CH₃)₂ oder Hydroxy,
- R⁸: steht jeweils unabhängig voneinander bevorzugt für C₁-C₆-Alkyl, Halogen, Cyano, Hydroxy, Amino, Nitro, C₂-C₆-Alkenyl, C₃-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₂-C₄-Alkoxyalkyl, C₁-C₄-Alkylamino, C₂-C₈-Dialkylamino, C₃-C₆-Cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Tri(C₁-C₂-alkyl)silyl,
- R⁸: steht jeweils unabhängig voneinander besonders bevorzugt für C₁-C₃-Alkyl, Cyclopropyl, Halogen, Hydroxy, C₁-C₃-Halogenalkyl, Halogencyclopropyl, C₁-C₂-Alkoxy, C₁-C₂-Halogenalkoxy oder C₂-C₃-Alkylcarbonyloxy,
- R⁸: steht jeweils unabhängig voneinander ganz besonders bevorzugt für Fluor, Chlor, Brom, Iod, Hydroxy, Methyl oder -OCH₃,
- R⁹: steht unabhängig voneinander bevorzugt für C₁-C₆-Alkyl, Halogen, Cyano, Hydroxy, Amino, Nitro, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₄-C₁₀-Cycloalkylalkyl, C₄-C₁₀-Alkylcycloalkyl, C₅-C₁₀-Alkylcycloalkylalkyl, C₃-C₆-Halogencycloalkyl, C₂-C₄-Alkoxyalkyl, C₁-C₄-Alkylamino, C₂-C₈-Dialkylamino, C₃-C₆-Cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₂-alkyl)silyl
oder
- R⁹: steht unabhängig voneinander bevorzugt für Phenyl oder Benzyl, das bis zu drei Substituenten enthalten kann, wobei die Substituenten unabhängig voneinander aus folgender Liste ausgewählt sind: C₁-C₃-Alkyl, Halogen, Cyano, Nitro, C₂-C₃-Alkenyl, C₂-C₃-Alkinyl, Cyclopropyl, C₁-C₃-Halogenalkyl, C₂-C₃-Halogenalkenyl, C₂-C₃-Halogenalkinyl, Halogencyclopropyl, C₁-C₂-Alkoxy oder C₁-C₂-Halogenalkoxy,
- R⁹: steht unabhängig voneinander besonders bevorzugt für C₁-C₆-Alkyl, Halogen, Cyano, Hydroxy, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Tri(C₁-C₂-alkyl)silyl
oder
- R⁹: steht unabhängig voneinander besonders bevorzugt für Phenyl, das bis zu drei Substituenten enthalten kann, wobei die Substituenten unabhängig voneinander aus folgender Liste ausgewählt sind: C₁-C₃-Alkyl, Halogen, Cyano, Nitro, C₂-C₃-Alkenyl, C₂-C₃-Alkinyl, C₁-C₃-Halogenalkyl, C₁-C₂-Alkoxy oder C₁-C₂-Halogenalkoxy,
- R⁹: steht unabhängig voneinander ganz besonders bevorzugt Chlor, Fluor, Brom, Iod, Cyano, - CH₃, -CH₂CH₃, -CH₂CH₂CH₃, -CH(CH₃)₂, -CH₂CH₂CH₂CH₃, -CH(CH₃)CH₂CH₃, - CH₂CH(CH₃)CH₃, -C(CH₃)₃, -CF₃, -CFH₂, -CF₂H, -CF₂CF₃, -CCl₃, Cyclopropyl, Cyclobutyl, Cyclopentyl, Cyclohexyl, -OCH₃, -OCH₂CH₃, -OCH₂CH₂CH₃, -OCH(CH₃)₂, - OCH₂CH₂CH₂CH₃, -OCH₂CH(CH₃)₂, -OCH(CH₃)CH₂CH₃, -OC(CH₃)₃, -OCF₃, -OCF₂H,-OCH₂CF₃, -OCF₂CF₃ SCH₃, -SCH₂CH₃, -SCH₂CH₂CH₃, -SCH(CH₃)₂, -SCH₂CH₂CH₂CH₃,-SCH₂CH(CH₃)₂, -SCH(CH₃)CH₂CH₃, -SC(CH₃)₃, -SCF₃, -SCF₂H, -SCH₂CF₃, -SCF₂CF₃, o-der Trimethylsilyl
oder
- R⁹: steht unabhängig voneinander ganz besonders bevorzugt für Phenyl, das bis zu drei Substituenten enthalten kann, wobei die Substituenten unabhängig voneinander aus folgender Liste: ausgewählt sind -CH₃, -CH₂CH₃, -CH₂CH₂CH₃, -CH(CH₃)₂, Chlor, Fluor, Brom, Iod, Cyano, -CH=CH₂, - CH=CHCH₃, -CH₂CH=CH₂, -C≡CH, -C≡CCH₃, -CH₂C≡CH, -CF₃, -CFH₂, -CF₂H, -CF₂CF₃, - CCl₃, -OCH₃, -OCH₂CH₃, -OCF₃, -OCF₂H, -OCH₂CF₃ oder -OCF₂CF₃
sowie agrochemisch wirksame Salze davon.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Ganz besonders bevorzugt sind solche Verbindungen der Formel (I), in welcher alle Reste jeweils die oben genannten ganz besonders bevorzugten Bedeutungen haben.

Insbesondere bevorzugt sind solche Verbindungen der Formel (I), in welcher alle Reste jeweils die oben genannten insbesondere bevorzugten Bedeutungen haben.

Weiterhin bevorzugt sind Verbindungen der Formel (I), in welcher X für Sauerstoff steht,
sowie agrochemisch wirksame Salze davon.

Weiterhin bevorzugt sind Verbindungen der Formel (I), in welcher Y für Sauerstoff steht,
sowie agrochemisch wirksame Salze davon.

Weiterhin bevorzugt sind Verbindungen der Formel (I), in welcher R¹ für Wasserstoff steht,
sowie agrochemisch wirksame Salze davon.

Weiterhin bevorzugt sind Verbindungen der Formel (I), in welcher R¹ für Fluor steht,
sowie agrochemisch wirksame Salze davon.

Weiterhin bevorzugt sind Verbindungen der Formel (I), in welcher Q für 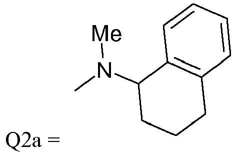 steht,
sowie agrochemisch wirksame Salze davon.

Weiterhin bevorzugt sind Verbindungen der Formel (I), in welcher Q für 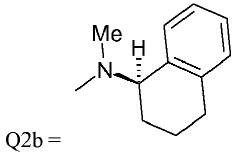 steht,
sowie agrochemisch wirksame Salze davon.

Weiterhin bevorzugt sind Verbindungen der Formel (I), in welcher Q für 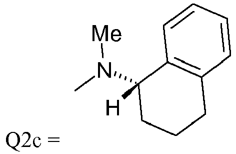 steht,
sowie agrochemisch wirksame Salze davon.

Je nach Art der oben definierten Substituenten weisen die Verbindungen der Formel (**I**) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (**I**) Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₁-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen**: Fluor, Chlor, Brom und Iod;
**Alkyl**: gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethyl-propyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkenyl**: ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl**: geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy**: gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methyl-propoxy;
**Alkylthio**: gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Alkoxycarbonyl**: eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl**: gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl**: gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl , 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl**: monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl**: geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Halogenalkoxy**: geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio**: geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl**: 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom**: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome**: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome**: 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom**: z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1, 3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome**: **:** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
**Heterocyclyl**: drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Verfahren und Zwischenprodukte

Die Bis(difluormethyl)pyrazol-Derivate der Formel (**I**) lassen sich auf unterschiedliche Weise herstellen. Im Folgenden sind die möglichen Verfahren zunächst schematisch dargestellt. Wenn nicht anders angegeben haben die Reste die oben angegebenen Bedeutungen.

### Verfahren A

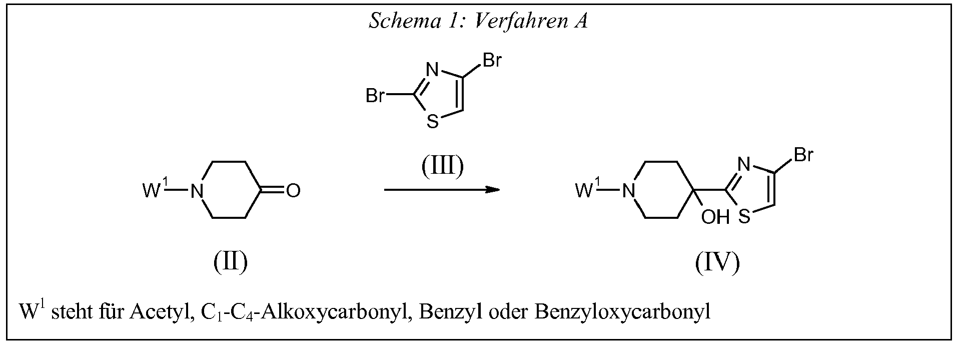

Eine Verbindung der allgemeinen Formel (**IV**) wird aus einer Verbindung der Formel (**III**) durch Halogen-Metall Austausch und folgender Zugabe einer Verbindung der allgemeinen Formel (**II**) erhalten (s. z.B. Org. Lett. 2004, 6, 3083-3085) (Schema 1).

Das Verfahren A wird in Gegenwart einer geeigneten metallorganischen Verbindung durchgeführt. Bevorzugte metallorganische Verbindungen sind Organolithium-Verbindungen (wie z.B. Butyllithium) oder Grignard Reagenzien (wie z.B. Isopropylmagnesiumhalogenid).

Das Verfahren A wird vorzugsweise unter Verwendung eines oder mehrerer Verdünnungsmittel durchgeführt. Es kommen vorzugsweise aprotische Lösungsmittel infrage, wie z.B. Dioxan, Glyme, Diethylether oder Tetrahydrofuran. Besonders bevorzugt ist die Verwendung von Tetrahydrofuran.

Die Reaktionstemperaturen können bei der Durchführung des Verfahrens A in einem größeren Bereich variiert werden. Im Falle der Halogen-Metall Austauschreaktionen arbeitet man im Allgemeinen bei Temperaturen von -120 °C bis +150 °C, vorzugsweise bei Temperaturen von -120 °C bis +60 °C, ganz besonders bevorzugt bei -120 °C bis 0 °C. Nach der Zugabe der Verbindung (**II**) arbeitet man bevorzugt bei -80 °C bis +50 °C.

Zur Durchführung des Verfahrens A setzt man pro Mol an Verbindung der Formel (**III**) im Allgemeinen 1 bis 2 Mol, vorzugsweise 1 Mol der metallorganischen Verbindung ein. Die Reaktionsdauer beträgt 1 bis 48 Stunden. Die Aufarbeitung erfolgt nach den üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren B

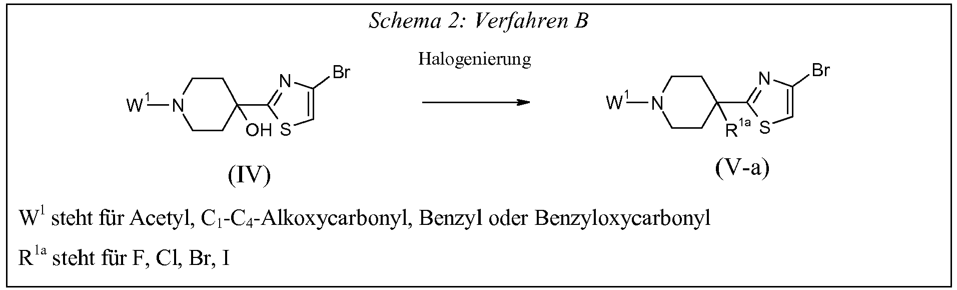

Das Verfahren B beschreibt die Herstellung von Verbindungen der Struktur (V-a), bei der R^{1a} = F, Cl, Br und I ist, durch Halogenierung von Verbindungen der Struktur (IV) (s. z.B. WO 2006/133216, WO 2004/108692, J. Med. Chem., 1991, 34, 108-122, EP-A 0 796 846, J. Antibiot., 1988, 41, 134-138, Bioorg. Med. Chem. Lett., 2008, 18, 5209-5212, Chem. Eur. J., 2004, 5640-5648, Russ. J. Org. Com., 2007, 50-55) (Schema 2).

Als Lösungsmittel können alle üblichen, unter den Reaktionsbedingungen inerten Lösungsmittel, verwendet werden oder die Reaktion kann in Mischungen von zwei oder mehreren dieser Lösungsmittel ausgeführt werden. Bevorzugt wird das Lösungsmittel Dichlormethan verwendet.

Als Halogenquelle kann z.B. Diethylaminoschwefeltrifluorid, Selectfluor, Deoxofluor, Thionylchlorid, PBr₃ und Methansulfonylchlorid verwendet werden.

Die Ausgangsmaterialien und das Halogenierungsmittel werden in äquimolaren Mengen eingesetzt. Das Halogenierungsmittel kann auch im Überschuss verwendet werden. Die Reaktion wird normalerweise bei Temperaturen von -80 °C bis +80 °C durchgeführt und vorzugsweise bei 0 °C bis +40 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion werden die Verbindungen (**V-a**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren C

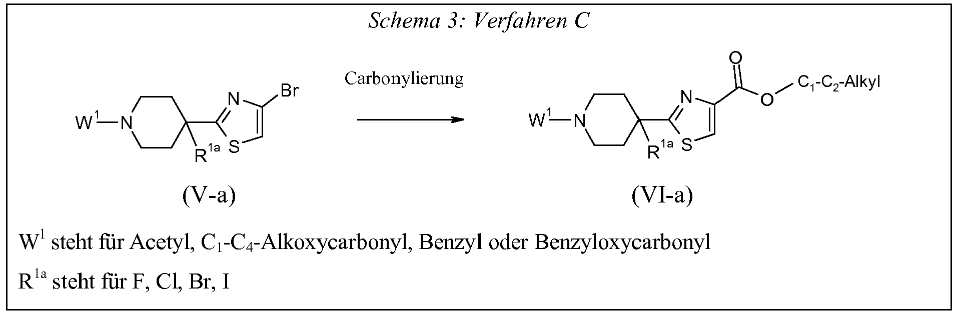

Eine Verbindung der allgemeinen Formel (**VI**-**a**) wird aus einer Verbindung der allgemeinen Formel (**V-a**) durch Metall-katalysierte Carbonylierung erhalten (s. z.B. Synthetic Communications, 1997, 515-520; Journal of Organic Chemistry, 2000, 7757-7763) (Schema 3).

Verfahren C wird in Gegenwart eines geeigneten Katalysators sowie gegebenenfalls in Gegenwart weiterer Co-Katalysatoren (z.B. CuI) und einer Base (z.B. Triethylamin oder Cs₂CO₃) durchgeführt. Die bevorzugten Katalysatoren sind Palladiumkatalysatoren (z.B. [(π-allyl)PdCl]₂, Pd(OAc)₂, PdCl₂(CH₃CN)₂, Pd(PPh₃)₄ oder PdCl₂(PPh₃)₂).

Verfahren C wird vorzugsweise unter Verwendung eines oder mehrer Verdünnungsmittel durchgeführt. Vorzugsweise kommen Alkohole (z.B. Methanol oder Ethanol) in Frage.

Die verwendete Katalysatormenge liegt bei 0,1-90mol% bezogen auf das Edukt, vorzugsweise werden 1-30mol% des Katalysators bezogen auf das Edukt verwendet. Die Reaktion kann bei Überdruck (1-1000 bar) und bevorzugt bei 1-5 bar Druck durchgeführt werden. Die Reaktion wird normalerweise bei Temperaturen von 0 °C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einer halben Stunde und 72 Stunden. Die Reaktion wird vorzugsweise bei 80 °C und einem CO-Druck von 3 bar durchgeführt.

Zur Durchführung des Verfahrens C setzt man pro Mol an Verbindung der Formel (**V-a**) im Allgemeinen 0,5 bis 10 Mol, vorzugsweise 1 bis 2 Mol einer Base. Die Aufarbeitung erfolgt nach üblichen Methoden.

Verbindungen der Formel (**VI-b**), in denen R^{1b} für Wasserstoff steht, sind bekannt und können aus kommerziell erhältlichen Vorstufen nach literaturbeschriebenen Vorschriften hergestellt werden (s. z.B. WO 2009/037357).

### Verfahren D

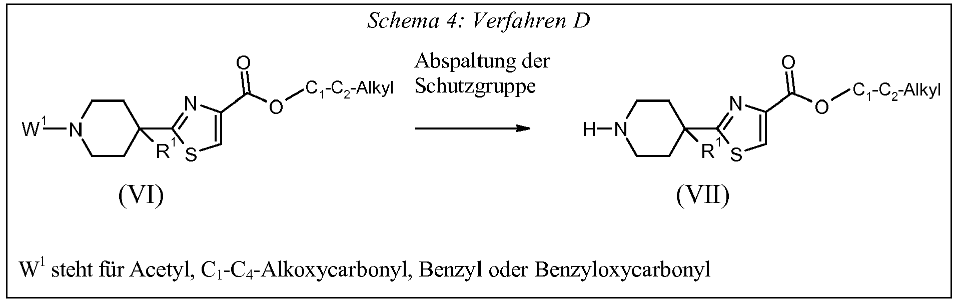

Eine Verbindung der Formel (**VI**) wird in eine Verbindung der Formel (**VII**) durch geeignete Methoden zur Entfernung von Schutzgruppen, die in der Literatur beschrieben sind ("Protective Groups in Organic Synthesis"; Third Edition; 1999; 494-653, und dort zitierte Literatur), überführt (Schema 4).

*tert*-Butoxycarbonyl und Benzyloxycarbonyl Schutzgruppen können im sauren Medium entfernt werden (z.B mit Salzsäure oder Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; 1999; S. 494-653).

Die Reaktion wird normalerweise bei Temperaturen von 0 ° C bis +150 °C durchgeführt und vorzugsweise bei Raumtemperatur, sie kann aber auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einer halben Stunde und 72 Stunden.

Nach Beendigung der Reaktion werden die Verbindungen (**VII**) von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel (**VII**) als Salz zu isolieren, z.B. als Salz der Salzsäure oder der Trifluoressigsäure.

### Verfahren E

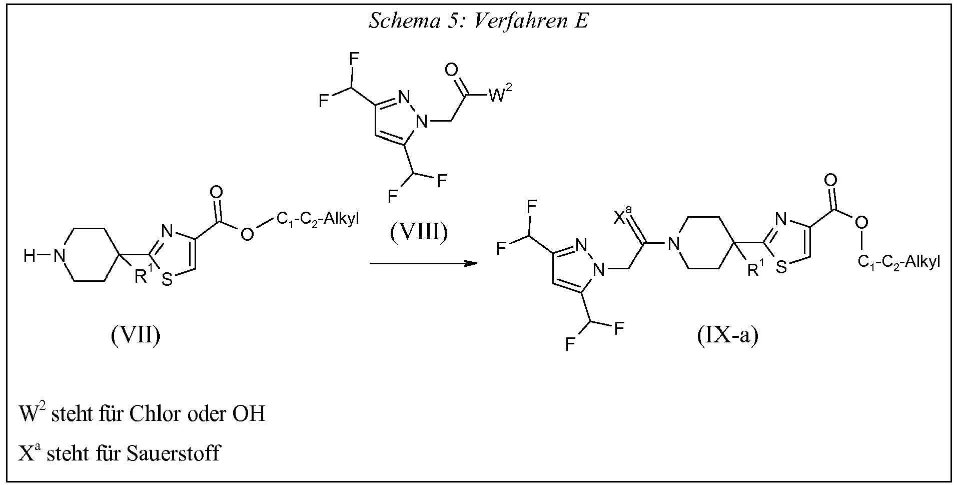

Eine Verbindung mit der allgemeinen Formel (**IX**-**a**) kann analog zu in der Literatur beschriebenen Vorschriften (s. z.B. WO 07/147336) synthetisiert werden, und zwar durch eine Kupplungsreaktion einer Verbindung mit der allgemeinen Formel (**VII**) mit einem Substrat der allgemeinen Formel (**VIII-a**) (W^{2a} = Chlor), gegebenenfalls in der Gegenwart eines Säurefängers / Base (Schema 5).

Verbindungen (**VIII-a**) (W^{2a} = Chlor) oder (**VIII-b**) (W^{2b} = OH) sind durch in der Literatur beschriebene Prozesse darstellbar (s. z.B. WO 2008/013622 und WO 2008/013925). Außerdem kann ein Substrat mit der Formel (**VIII**-**a**) (W^{2a} = Chlor) aus der entsprechenden Säure mit der Formel (**VIII-b**) (W^{2b} = OH) durch Chlorierung unter Verwendung literaturbekannter Prozesse dargestellt werden (z.B. Tetrahedron 2005, 61, 10827-10852, und darin zitierte Literatur).

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig Base, Triethylamin oder kommerziell erhältliche polymere Säurefänger) wird im Verhältnis zum Startmaterial der allgemeinen Formel (**VII**) verwendet. Ist das Startmaterial ein Salz, werden wenigstens zwei Äquivalente des Säurefängers benötigt.

Die Reaktion wird normalerweise bei Temperaturen von 0 °C bis 100 °C durchgeführt und vorzugsweise bei 20 °C bis 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion werden die Verbindungen (**IX**-**a**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung der Formel (**IX**-**a**) auch aus der entsprechenden Verbindung der Formel (**VII**) mit einem Substrat der Formel (**VIII**-**b**) (W^{2b} = OH) in Gegenwart eines Kupplungsreagenzes synthetisiert werden (analog zu in der Literatur beschriebenen Vorschriften, z.B. Tetrahedron 2005, 61, 10827-10852, und darin zitierte Referenzen).

Geeignete Kupplungsreagenzien sind beispielsweise Peptidkupplungsreagenzien (z.B. N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-l-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Das bevorzugte Lösungsmittel ist Dichlormethan.

Die Reaktion wird normalerweise bei Temperaturen von 0 °C - 100 °C durchgeführt und vorzugsweise bei 0 °C - 30 °C, sie kann aber auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion, werden die Verbindungen (**IX**-**a**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren F

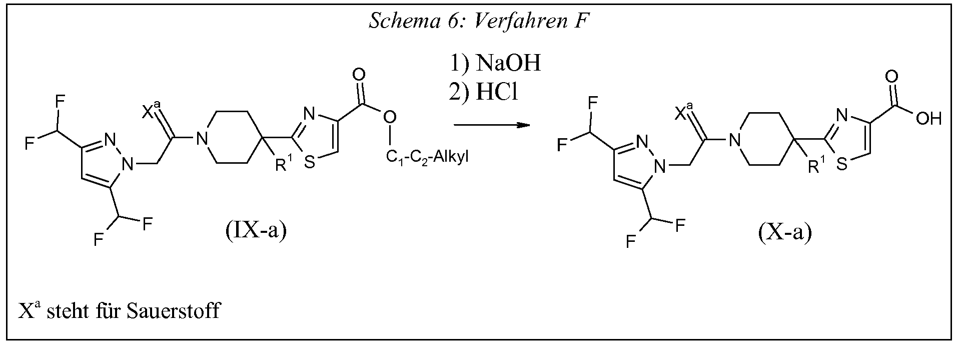

Die Carbonsäure der Formel (**X-a**) kann durch Verseifung des entsprechenden C₁-C₂-Alkylesters der Formel (**IX**-**a**) dargestellt werden. Zum Beispiel kann die Methode, die in WO 2007/014290 beschrieben wird, verwendet werden (Schema 6).

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff) und halogenierte aromatische Kohlenwasserstoffe (z.B. Chlorbenzol, Dichlorbenzol) verwendet werden oder die Reaktion kann in Mischungen von zwei oder mehreren dieser Lösungsmittel ausgeführt werden.

Geeignete Alkalimetallhydroxide sind beispielsweise LiOH, NaOH oder KOH, gewöhnlich in der Gegenwart von Wasser zusammen mit einem Cosolvens, bevorzugt THF und/oder Methanol, um das Lösen des Esters zu vereinfachen. Das Startmaterial und das Alkalimetallhydroxid werden in äquimolaren Mengen verwendet, aber das Alkalimetallhydroxid kann gegebenenfalls auch im Überschuss verwendet wurden. Das entstehende Carboxylatsalz wird in die freie Säure durch das Behandeln mit einem geringen Überschuss an Mineralsäuren, wie zum Beispiel Salzsäure oder Schwefelsäure überführt.

Die Reaktion wird normalerweise bei Temperaturen von 0 °C - 60 °C durchgeführt, sie kann aber auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion werden die Verbindungen (**X-a**) von der Reaktionsmischung durch eine der üblichen Trenntechniken getrennt. Falls notwendig werden die Verbindungen durch Umkristallisation, Destillation oder Chromatographie gereinigt.

### Verfahren G

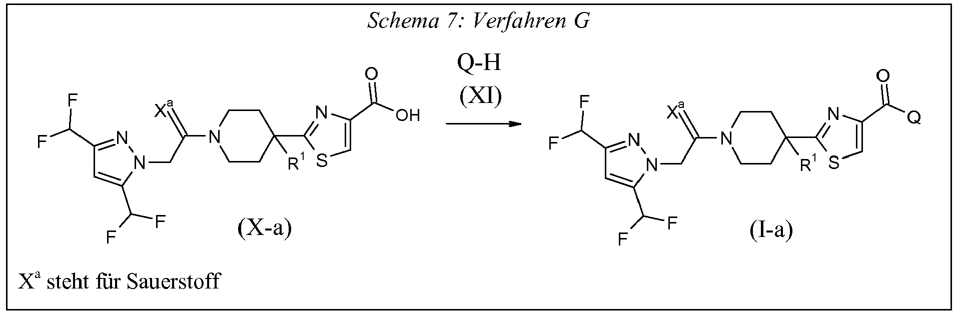

Eine Verbindung mit der Formel (**I-a**) kann aus der entsprechenden Verbindung der Formel (**X-a**) mit einem Substrat der Formel (**XI**) in der Gegenwart eines Kupplungsreagenzes synthetisiert werden (analog zu in der Literatur beschriebenen Vorschriften, z.B. Tetrahedron 2005, 61, 10827-10852, und darin zitierte Referenzen).

Geeignete Kupplungsreagenzien sind beispielsweise Peptidkupplungsreagenzien (wie etwa N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Die bevorzugten Lösungsmittel sind N,N-Dimethylformamid und Dichlormethan.

Die Reaktion wird normalerweise bei Temperaturen von 0 °C - 100 °C durchgeführt und vorzugsweise bei 0 °C - 30 °C, sie kann aber auch bei Rückflusstemperatur der Reaktionsmischung durchgeführt werden. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion, werden die Verbindungen (**I-a**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung der Formel (**I-a**) auch ausgehend von der Verbindung der Formel (**X-a**) durch eine zweistufige Transformation synthetisiert werden (unter Verwendung literaturbekannter Prozesse, z.B. Tetrahedron 2005, 61, 10827-10852, und darin zitierte Literatur), gegebenenfalls in der Gegenwart eines Säurefängers / Base. Typischerweise wird eine Verbindung der Formel (**X-a**) zunächst in das korrespondierende Säurehalogenid oder -sulfonat überführt, dann erfolgt eine Kupplungsreaktion mit einem Substrat der Formel (**XI**).

Substrate mit der allgemeinen Formel (**XI**) sind kommerziell erhältlich oder durch in der Literatur beschriebene Prozesse darstellbar (siehe z.B. "The Chemistry of Functional groups"; "The Chemistry of the Thiol Group"; John Wiley & Sons, 1974, 163-269, und darin zitierte Referenzen; "The Chemistry of Functional groups"; "Supplement F2: The Chemistry of amino, nitroso, nitro and related groups"; John Wiley & Sons, und darin zitierte Referenzen; "Science of Synthesis"; "Alcohols", Volume 36, Thieme, 2008 und darin zitierte Referenzen; "Science of Synthesis"; "Amines and Ammonium Salts", Volume 40a, Thieme, 2008, und darin zitierte Referenzen).

Bei der Durchführung des erfindungsgemäßen Verfahrens G können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden, oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig-Base, Triethylamin oder kommerziell erhältliche polymere Säurefänger) im Verhältnis zum Startmaterial der allgemeinen Formel (**XI**) wird verwendet. Ist das Startmaterial ein Salz, werden wenigstens zwei Äquivalente des Säurefängers benötigt.

Die Reaktion wird normalerweise bei Temperaturen von 0 °C - 100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion werden die Verbindungen (**I-a**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren H

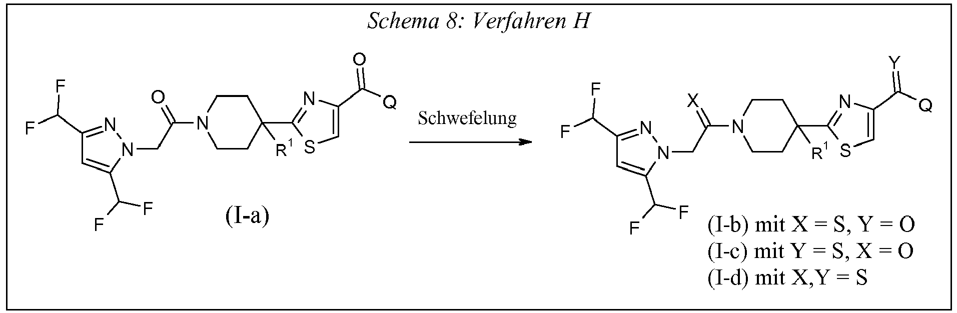

Eine Möglichkeit Verbindungen der Formel (**I-b**), (**I-c**) oder (**I-d**) aus entsprechenden Verbindungen (**I-a**) darzustellen, ist in Schema 8 gezeigt.

Als Lösungsmittel können alle üblichen, unter den Reaktionsbedingungen inerten Lösungsmittel, wie z.B. Alkohole (z.B. Methanol, Ethanol, Propanol), zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester) und Amide (z.B. N,N-Dimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Chloroform und 1,2-Dimethoxyethan.

Geeignete Schwefelungsreagenzien sind beispielsweise Lawesson's Reagenz (s. Tetrahedron 1986, 42, 6555-6564, Tetrahedron Lett. 1993, 46, 7459-7462) und Phosphorpentasulfid.

Das Schwefelungsreagenz wird im Verhältnis zum Startmaterial im Überschuss eingesetzt (mehr als zwei Äquivalente).

Die Reaktion wird normalerweise bei Temperaturen von 0 °C - 150 °C durchgeführt und vorzugsweise bei 0 °C - 100 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Die Reaktion verläuft bei einem Überschuss an Schwefelungsreagenz nicht selektiv. Man erhält eine Mischung der Produkte (**I-b**), (**I-c**) und (**I-d**), die nach Beendigung der Reaktion durch eine der üblichen Trenntechniken, z.B. Chromatographie, getrennt werden.

Die vorliegende Erfindung betrifft weiterhin ein Pflanzenschutzmittel zum Bekämpfen unerwünschter Pilze umfassend wenigstens eines der Bis(difluormethyl)pyrazol Derivate der Formel (I). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man erfindungsgemäß Bis(difluormethyl)pyrazol Derivate der Formel (**I**) auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl-dispergierbare Pulver, Öl-mischbare fließfähige Konzentrate, Öl-mischbare Flüssigkeiten, Schäume, Pasten, Pestizid-ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur-und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Pilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European com borer) und/oder Western Com Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke fungizide Wirkung auf und können zur Bekämpfung von unerwünschten Pilzen im Pflanzenschutz und im Materialschutz eingesetzt werden.

Die erfindungsgemäßen Bis(difluormethyl)pyrazol-Derivate lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht werden.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Olenceae sp., Actinidaceae sp., Lauraceae sp., Musaceae sp.* (beispielsweise Bananenbäume und - plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Poaceae sp.* (z.B. Zuckerrohr), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befmdlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp.*, die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Aceto-hydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum B eispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor pilzlicher Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha;
Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora.

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Organismen, die einen Abbau oder eine Veränderung der technischen Materialien bewirken können, seien Pilze genannt. Vorzugsweise wirken die erfindungsgemäßen Wirkstoffe gegen Pilze, insbesondere Schimmelpilze, Holz verfärbende und Holz zerstörende Pilze (Basidiomyceten). Es seien beispielsweise Pilze der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die aufgeführten Pflanzen können besonders vorteilhaft erfindungsgemäß mit den Bis(difluormethyl)pyrazol Derivate der Formel (I) oder den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Herstellung und die Verwendung der erfindungsgemäßen Wirkstoffe der Formeln (I) geht aus den folgenden Beispielen hervor. Die Erfindung ist jedoch nicht auf diese Beispiele beschränkt.

**Allgemeines:** Wenn nicht anders angegeben, wurden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Hexan durchgeführt.

### Herstellung von Verbindungen der Formel (I)

### Verfahren A

### tert-Butyl-4-(4-brom-1,3-thiazol-2-yl)-4-hydroxypiperidin-1-carboxylat (IV-1)

Zu einer Lösung von 2,4-Dibrom-1,3-thiazol (8.8 g) in Dichloromethan (180 mL) wurde bei -78 °C unter Argonatmosphäre *n*-Butyllithium (1.6 M in Tetrahydrofuran, 25 mL) getropft. Das Reaktionsgemisch wurde 20 Minuten bei -78 °C gerührt und dann wurde tert-Butyl-4-oxopiperidin-1-carboxylat zugegeben. Das Gemisch wurde bei Raumtemperatur für 30 Minuten gerührt. Das Reaktionsgemisch wurde anschließend mit gesättigter Ammoniumchloridlösung bei -30 °C versetzt und die wässrige Phase abgetrennt. Nach Extraktion der wässrigen Phase mit Dichloromethan wurden die vereinigten organischen Phasen über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-hydroxypiperidin-1-carboxylat (15.3 g).
¹H NMR (DMSO-d₆): δₚₚₘ : 1.43 (s, 9H), 1.70 (d, 2H), 1.88 (ddd, 2H), 3.11 (bs, 2H), 3.83 (d, 2H), 6.31 (s, 1H), 7.72 (s, 1H)
logP (HCOOH): 2.74
MS (ESI): 363 und 365 ([M+H]⁺)

### Verfahren B

### tert-Butyl-4-(4-brom-1,3-thiazol-2-yl)-4-fluorpiperidin-1-carboxylat (Va-1)

*tert*-Butyl-4-(4-brom-1,3-thiazol-2-yl)-4-hydroxypiperidin-1-carboxylat (17.7 g) wurde unter Argonatmosphäre bei 0 °C in Dichloromethan in einem Polyethylen-Kolben vorgelegt und Diethylaminoschwefeltrifluorid (DAST) (7.08 mL) wurde zugetropft. Die Kühlung wurde entfernt. Nachdem über Nacht gerührt wurde, wurde das Reaktionsgemisch mit gesättigter wässriger Natriumhydrogencarbonatlösung versetzt und anschließend mit Dichlromethan extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-fluorpiperidin-1-carboxylat (18.0 g).
¹H NMR (DMSO-d₆): δₚₚₘ : 1.42 (s, 9H), 2.13-2.00 (m, 4H), 3.14 (bs, 2H), 3.95-3.87 (m, 2H), 7.95 (s, 1H)
logP (HCOOH): 3.94
MS (ESI): 309 und 311 ([M-C(CH₃)₃+2H]⁺)

### Verfahren C

### tert-Butyl-4-[4-(ethoxycarbonyl)-1,3-thiazol-2-yl]-4-fluorpiperidin-1-carboxylat (VIa-1)

*tert*-Butyl-4-(4-brom-1,3-thiazol-2-yl)-4-fluorpiperidin-1-carboxylat (29 g) wurde in Ethanol (150 mL) gelöst und bei 70 °C unter einem Druck von 3 bar CO für 48 Stunden in Gegenwart von PdCl₂(PPh)₂ (2.79 g) und Triethylamin (77 mL) gerührt. Der Katalysator wurde durch Filtration über Celite entfernt und unter vermindertem Druck eingeengt. Nach chromatografischer Reinigung erhielt man *tert-*Butyl-4-[4-(ethoxycarbonyl)-1,3-thiazol-2-yl]-4-fluorpiperidin-1-carboxylat (380 mg).
logP(pH2.7):3.47
¹H NMR (DMSO-d₆, 400 MHz): δₚₚₘ : 1.31 (t, 3H), 1.42 (s, 9H), 2.00-2.20 (m, 4H), 3.13 (bs, 2H), 3.90-3.98 (m, 2H), 4.32 (q, 2H), 8.60 (s, 1H)
MS (ESI): 303 ([M-C(CH₃)₃+2H]⁺)

### Verfahren D

### 4-[4-(Ethoxycarbonyl)-1,3-thiazol-2-yl]-4-fluorpiperidiniumchlorid (VII-1)

Zu *tert*-Butyl-4-[4-(ethoxycarbonyl)-1,3-thiazol-2-yl]-4-fluorpiperidin-1-carboxylat (8.80 g) wurde unter Argonatmosphäre, bei 0 °C eine Lösung von Chlorwasserstoff in Dioxan (4 M, 92 mL) gegeben. Das Gemisch wurde bei 0 °C gerührt und danach langsam auf Raumtemperatur erwärmt. Nach Rühren für 24 Stunden wurde die überschüssige Säure und das Lösungsmittel unter vermindertem Druck entfernt. Man erhielt 4-[4-(Ethoxycarbonyl)-1,3-thiazol-2-yl]-4-fluorpiperidiniumchlorid (7.70 g).
logP (pH2.7): 0.39
¹H NMR (DMSO-d₆, 400 MHz): δₚₚₘ : 1.31 (t, 3H), 2.30-2.60 (m, 4H), 3.10-3.21 (m, 2H), 3.32-3.42 (2H), 4.32 (q, 2H), 8.64 (s, 1H), 9.20 (bs, 1H)
MS (ESI): 259 ([MCl]⁺)

### Verfahren E

### Ethyl-2-(1-{[3,5-bis(difluormethyl)-1H-pyrazol-1-yl] acetyl}-4-fluorpiperidin-4-yl)-1,3-thiazol-4-carboxylat (IXa-1)

Zu einer Lösung von [3,5-Bis-(difluormethyl)-1H-pyrazol-1-yl]essigsäure (230 mg) in Dichlormethan (5 mL) wurde bei 0 °C Oxalylchlorid (0.89 mL) und ein Tropfen N,N-Dimethylformamid gegeben. Das Reaktionsgemisch wurde bei Raumtemperatur für 24 Stunden gerührt. Das Lösungsmittel und das überschüssige Reagenz wurden unter vermindertem Druck entfernt. Der feste Rückstand wurde dann erneut in Dichlormethan gelöst und tropfenweise bei 0 °C zu einer Lösung von 4-[4-(Ethoxycarbonyl)-1,3-thiazol-2-yl]-4-fluorpiperidiniumchlorid (767 mg) und N,N-Diisopropylethylamin (1.8 mL) in Dichlormethan (5 mL) gegeben. Nachdem für 2 Stunden gerührt wurde, wurde die Reaktionslösung mit konzentrierter Ammoniumchloridlösung versetzt, die wässrige Phase abgetrennt und mit Essigsäure-ethylester extrahiert. Die vereinigten organischen Phasen wurden über wasserfreiem Natriumsulfat getrocknet und eingeengt. Man erhielt Ethyl-2-(1-{[3,5-bis(difluormethyl)-1H-pyrazol-1-yl]acetyl}-4-fluorpiperidin-4-yl)-1,3-thiazol-4-carboxylat (1.02g).
logP (pH2.7): 2.82
¹H NMR (DMSO-d₆, 400 MHz): δₚₚₘ : 1.31 (t, 3H), 2.05-2.21 (m, 3H), 2.38-2.51 (m, 1H), 3.03-3.08 (m, 1H), 3.42-3.48 (m, 1H), 3.90-3.95 (m, 1H), 4.27-4.33 (m, 3H), 5.42 (d, 1H). 5.53 (d, 1H), 6.93 (s, 1H), 7.05 (t, 1H), 7.20 (t, 1H), 8.64 (s, 1H)
MS (ESI): 467 ([M+H]⁺)

### Verfahren F

**2-(1-{[3,5-Bis(difluormethyl)-1H-pyrazol-1-yl]acetyl}-4-fluorpiperidin-4-yl)-1,3-thiazol-4-carbonsäure (Xa-1)**

Zu einer Lösung von Ethyl-2-(1-{[3,5-bis(difluormethyl)-1H-pyrazol-1-yl]acetyl}-4-fluorpiperidin-4-yl)-1,3-thiazol-4-carboxylat (300 mg) in Tetrahydrofuran (3 mL) und Wasser (1 mL) wurde bei Raumtemperatur Lithiumhydroxidmonohydrat (40 mg) gegeben. Das Gemisch wurd 3 Stunden bei Raumtemperatur gerührt und dann mit eiskalter 1N HCl-Lösung versetzt. Die wässrige Phase wurde mit Ethylacetat extrahiert und dann die vereinigten organischen Phasen mit Natriumsulfat getrocknet. Der Feststoff wurde abfiltriert und das Lösungsmittel abdestilliert. Man erhielt 2-(1-{[3,5-Bis(difluormethyl)-1H-pyrazol-1-yl]acetyl}-4-fluorpiperidin-4-yl)-1,3-thiazol-4-carbonsäure (230 mg).
logP(pH2.7): 1.96
¹H NMR (DMSO-d₆, 400 MHz): δₚₚₘ : 2.00-2.60 (m, 4H), 3.02-3.13 (m, 1H), 3.40-3.51 (m, 1H), 3.86-3.99 (m, 1H), 4.22-4.30 (m, 1H), 5.40 (d, 1H), 5.50 (d, 1H), 6.91 (s, 1H), 7.03 (t, 1H), 7.19 (t, 1H), 8.54 (s, 1H), 13.10 (bs, 1H)
MS (ESI): 439 ([M+H]⁺)

### Verfahren G

### (±)-2-(1-{[3,5-Bis(difluormethyl)-1H-pyrazol-1-yl]acetyl}-4-fluorpiperidin-4-yl)-N-methyl-N-(1,2,3,4-tetrahydronaphthalen-1-yl)-1,3-thiazol-4-carboxamid (Ia-4)

2-(1-{[3,5-Bis(difluormethyl)-1H-pyrazol-1-yl]acetyl}-4-fluorpiperidin-4-yl)-1,3-thiazol-4-carbonsäure (2 g), (±)-N-Methyl-1,2,3,4-tetrahydronaphthalen-1-amin (736 mg) und Hünigbase (1.59 mL) wurden in Dichlormethan (20 mL) gelöst und danach wurde Brom-tris-pyrrolidinophosphoniumhexafluorophosphat (2.34 g) zugegeben. Die Reaktionsmischung wurde über Nacht bei Raumtemperatur gerührt. Nach Entfernen des Lösungsmittels unter vermindertem Druck wurde der Rückstand chromatografisch gereinigt. Man erhielt (±)-2-(1-{[3,5-Bis(difluormethyl)-1H-pyrazol-1-yl]acetyl}-4-fluorpiperidin-4-yl)-N-methyl-N-(1,2,3,4-tetrahydronaphthalen-1-yl)-1,3-thiazol-4-carboxamid (1.70 g).
logP (pH2.7): 3.56
¹H NMR (DMSO-d₆, 400 MHz): δₚₚₘ : 1.50-2.85 (m, 13H), 3.10-3.25 (m, 1H), 3.40-3.55 (m, 1H), 3.80-3.94 (m, 1H), 4.03-4.28 (m, 1H), 5.17-5.53 und 5.77-5.83 (m, 3H), 6.90 (s, 1H), 7.00-7.40 (m, 5H), 8.28 und 8.32 (s, 1H)
MS (ESI): 439 ([M+H]⁺)

### (-)-2-(1-{[3,5-Bis(difluormethyl)-1H-pyrazol-1-yl]acetyl}-4-fluorpiperidin-4-yl)-N-methyl-N-(1,2,3,4-tetrahydronaphthalen-1-yl)-1,3-thiazol-4-carboxamid (I-5) und (+)-2-(1-{[3,5-Bis(difluormethyl)-1H-pyrazol-1-yl]acetyl}-4-fluorpiperidin-4-yl)-N-methyl-N-(1,2,3,4-tetrahydronaphthalen-1-yl)-1,3-thiazol-4-carboxamid (I-6)

Die beiden Enantiomere von (±)-2-(1-{[3,5-Bis(difluormethyl)-1H-pyrazol-1-yl]acetyl}-4-fluorpiperidin-4-yl)-N-methyl-N-(1,2,3,4-tetrahydronaphthalen-1-yl)-1,3-thiazol-4-carboxamid (**I-4**) wurden mittels chiraler präparativer HPLC isoliert.

### Beispiele

**Tabelle 1**

| | | | |
|---|---|---|---|
| 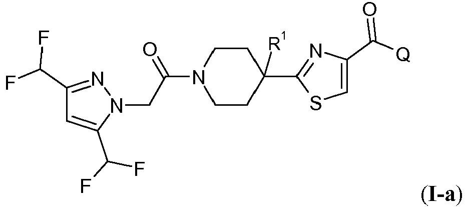 | | | |

| Beispiel | R¹ | Q | logP |
|---|---|---|---|
| I-a-1 | Wasserstoff | 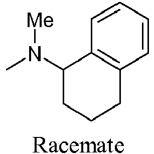 | 3,41 |
| I-a-2 | Wasserstoff | 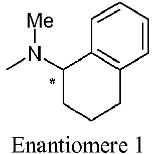 | - |
| I-a-3 | Wasserstoff | 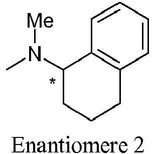 | - |
| I-a-4 | Fluor | 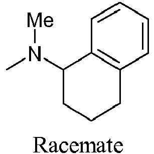 | 3,56 |
| I-a-5 | Fluor | 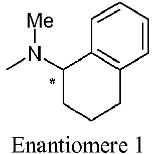 | - |
| I-a-6 | Fluor | 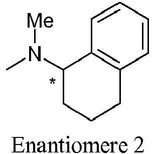 | - |

Die Messung der logP Werte erfolgte gemäß EEC Directive 79/831 Annex V.A8 durch HPLC (High Performance Liquid Chromatography).

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).Die lambda-maX-Werte wurden an Hand der UV-Spektren von 200 nm bis 400 nm in den Maxima der chromatographischen Signale ermittelt.

### Spezifischer Drehwert

Wellenlänge: 589 nm; Temperatur: 20,0 °C; Lösungsmittel: Methanol

| **Beispiel** | **Conzentration in MeOH** | **Spezifischer Drehwert** |
|---|---|---|
| I-a-2 | 95,8 mg / 10 mL | -57,2 |
| I-a-3 | 98,3 mg / 10 mL | +60,1 |
| I-a-5 | 102,2 mg / 10 mL | -54,1 |
| I-a-6 | 107,0 mg / 10 mL | +49,8 |

**NMR-Daten**

| **Beispiel** | **NMR-Daten** |
|---|---|
| I-a-1 | 1.50-2.15 (m, 8H), 2.51-2.88 (m+s, 6H), 3.27-3.40 (m, 2H), 3.89-3.97 (m, 1H), 4.23-4.36 (m, 1H), 5.32-5.38 und 5.80-5.83 (m, 3H), 6.90 (s, 1H), 6.93-7.26 (m, 6H), 8.08 und 8.12 (s, 1H) |
| I-a-4 | 1.50-2.85 (m, 13H), 3.10-3.25 (m, 1H), 3.40-3.55 (m, 1H), 3.80-3.94 (m, 1H), 4.03-4.28 (m, 1H), 5.17-5.53 und 5.77-5.83 (m, 3H), 6.90 (s, 1H), 7.00-7.40 (m, 6H), 8.28 und 8.32 (s, 1H) |

Die chemischen NMR-Verschiebungen in ppm wurden bei 400 MHz im Lösungsmittel DMSO-d₆ mit Tetramethylsilan als internem Standard gemessen.

### Verwendungsbeispiele

### Beispiel A

### Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen **I-a-1, I-a-2** und **I-a-3** bei einer Konzentration an Wirkstoff von 100ppm einen Wirkungsgrad von 70% oder mehr.

### Beispiel B

### Plasmopara-Test (Rebe) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **I-a-1, I-a-2** und **I-a-3** bei einer Konzentration an Wirkstoff von 100ppm einen Wirkungsgrad von 70% oder mehr.

## Patentansprüche

1. Verbindungen der Formel (I), 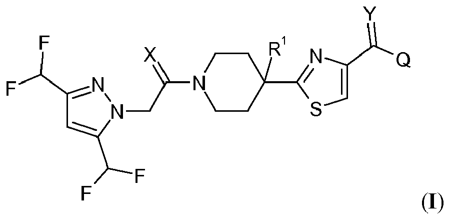 in welcher die Symbole folgende Bedeutungen haben:
X steht für Sauerstoff oder Schwefel,
R¹ steht für Wasserstoff oder Halogen,
Y steht für Sauerstoff oder Schwefel,
Q steht für -NR²R³,
R² steht für Wasserstoff, C₁-C₃-Alkyl, C₂-C₃-Alkenyl, C₂-C₃-Alkinyl, C₄-C₁₀-Cycloalkylalkyl, C₄-C₁₀-Alkylcycloalkyl, C₅-C₁₀-Alkylcycloalkylalkyl, C₁-C₃-Halogenalkyl, C₂-C₃-Halogenalkenyl, Cyano, Hydroxy, C₁-C₃-Alkoxy, C₂-C₃-Alkoxyalkyl, C₁-C₃-Hydroxyalkyl, C₂-C₃-Alkylcarbonyl, C₂-C₃-Alkoxycarbonyl, C₂-C₃-Alkylaminocarbonyl oder C₃-C₅-Dialkylaminocarbonyl,
R³ steht für ein 8- bis 11-gliedriges, unsubstituiertes oder substituiertes, gesättigtes oder teilweise gesättigtes bicyclisches Ringsystem oder steht für ein 10- bis 15-gliedriges, unsubstituiertes oder substituiertes, gesättigtes oder teilweise gesättigtes tricyclisches Ringsystem; jedes Ringsystem kann gegebenenfalls ein bis drei Heteroatome enthalten (bis zu ein Sauerstoffatom, bis zu ein Schwefelatom und bis zu drei Stickstoffatomen) und gegebenenfalls ein bis drei Ringglieder, die aus der Gruppe C(=O), C(=S), S(=O) oder S(=O)₂ ausgewählt werden können
oder
R² und R³ bilden zusammen mit dem Stickstoffatom, an das sie gebunden sind, einen 5- oder 6-gliedrigen, unsubstituierten oder substituierten, gesättigten oder teilweise gesättigten Heterocyclylrest
oder
R³ steht für -CR⁴R⁵R⁶,
R⁴ steht für Wasserstoff, C₁-C₆-Alkyl, C₂-C₆-Alkenyl, C₂-C₆-Alkinyl, C₃-C₆-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, Cyano, Nitro, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylaminocarbonyl, C₃-C₈-Dialkylaminocarbonyl oder C₃-C₆-Trialkylsilyl,
R⁵ steht für unsubstituierts oder substituierts Phenyl, Benzyl, Naphthalenyl, C₃-C₆-Cycloalkyl, C₃-C₆-Cycloalkenyl oder einen 5- oder 6-gliedrigen Heteroarylrest,
R⁶ steht für Wasserstoff, C₁-C₄-Alkyl, C₂-C₄-Alkenyl, C₂-C₄-Alkynyl, C₃-C₄-Cycloalkyl, C₄-C₁₀-Cycloalkylalkyl, C₄-C₁₀-Alkylcycloalkyl, C₅-C₁₀-Alkylcycloalkylalkyl, C₁-C₄-Halogenalkyl, C₂-C₄-Halogenalkenyl, C₂-C₄-Halogenalkinyl, C₃-C₄-Halogencycloalkyl oder C₂-C₄-Alkoxyalkyl,
oder
R² und R⁴ bilden zusammen mit den Atomen, an die sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu zwei Stickstoffatomen),
oder
R² und R⁵ bilden zusammen mit den Atomen, an die sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu zwei Stickstoffatomen),
oder
R⁴ und R⁶ bilden zusammen mit dem Kohlenstoff, an den sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu ein Stickstoffatom),
oder
R⁴ und R⁵ bilden zusammen mit dem Kohlenstoff, an den sie gebunden sind, ein 5- bis 7-gliedriges, unsubstituiertes oder substituiertes Ringsystem, bestehend aus zwei bis sieben Kohlenstoffatomen und gegebenfalls ein bis drei Heteroatomen (bis zu ein Sauerstoffatom, bis zu ein Schwefelatom und bis zu ein Stickstoffatom),
sowie agrochemisch wirksame Salze davon.

2. Verbindungen der Formel (I) nach Anspruch 1,
in welcher die Symbole folgende Bedeutungen haben,
X steht für Sauerstoff,
R¹ steht für Wasserstoff oder Fluor,
Y steht für Sauerstoff,
Q steht für, 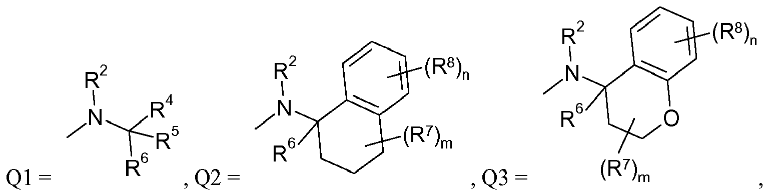 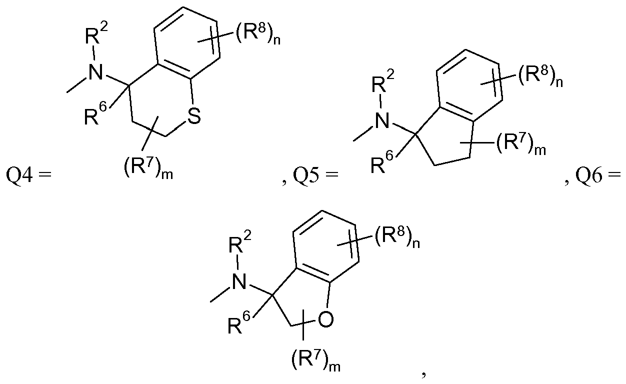 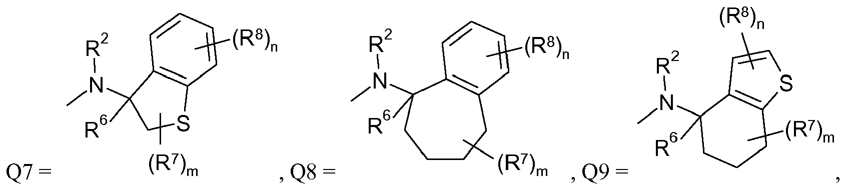 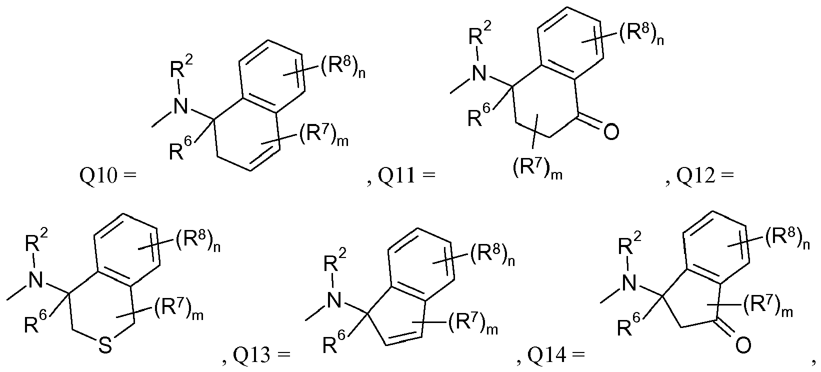 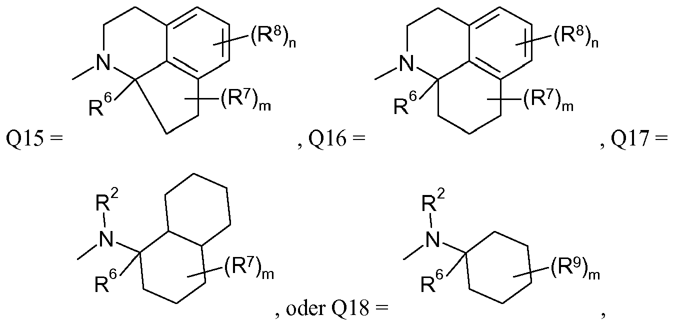
m steht für 0, 1 oder 2,
n steht für 0, 1 oder 2,
R² steht für Wasserstoff oder C₁-C₃-Alkyl,
R⁴ steht für Wasserstoff, C₁-C₄-Alkyl, C₃-C₆-Cycloalkyl, C₂-C₄-Alkoxyalkyl, C₁-C₆-Halogenalkyl,
R⁵ steht für ein unsubstituierten oder substituierten Phenyl, Benzyl oder C₃-C₆-Cycloalkyl,
R⁶ steht für Wasserstoff oder C₁-C₄-Alkyl, C₃-C₄-Cycloalkyl, C₂-C₄-Alkoxyalkyl, C₁-C₄-Halogenalkyl,
R⁷ steht jeweils unabhängig voneinander für Wasserstoff, C₁-C₄-Alkyl, Halogen, Cyano, Hydroxy, Amino, Nitro, C₂-C₄-Alkenyl, C₃-C₄-Alkinyl, C₁-C₄-Halogenalkyl, C₂-C₄-Halogenalkenyl, C₂-C₄-Halogenalkinyl, C₃-C₆-Cycloalkyl, C₃-C₆-Halogen-cycloalkyl, C₂-C₄-Alkoxyalkyl, C₁-C₄-Alkylamino, C₂-C₆-Dialkylamino, C₃-C₆-Cycloalkylamino, C₂-C₄-Alkylcarbonyl, C₂-C₄-Alkoxycarbonyl, C₂-C₄-Alkylaminocarbonyl, C₂-C₄-Alkylaminocarbonyloxy, C₃-C₆- Dialkylaminocarbonyl, C₁-C₄-Hydroxyalkyl, C₁-C₄-Alkoxy, C₁-C₄-Halogenalkoxy, C₂-C₄-Alkylcarbonyloxy, C₂-C₄-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Tri(C₁-C₂-alkyl)silyl,
R⁸ steht jeweils unabhängig voneinander für C₁-C₆-Alkyl, Halogen, Cyano, Hydroxy, Amino, Nitro, C₂-C₆-Alkenyl, C₃-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₂-C₄-Alkoxyalkyl, C₁-C₄-Alkylamino, C₂-C₈-Dialkylamino, C₃-C₆-Cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C1-C₄-Alkylsulfinyl, C₁-C₄-Halogenalkylsulfinyl, C₁-C₄-Alkylsulfonyl, C₁-C₄-Halogenalkylsulfonyl oder Tri(C₁-C₂-alkyl)silyl,
R⁹ steht unabhängig voneinander für C₁-C₆-Alkyl, Halogen, Cyano, Hydroxy, Amino, Nitro, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₄-C₁₀-Cycloalkylalkyl, C₄-C₁₀-Alkylcycloalkyl, C₅-C₁₀-Alkylcycloalkylalkyl, C₃-C₆-Halogencycloalkyl, C₂-C₄-Alkoxyalkyl, C₁-C₄-Alkylamino, C₂-C₈-Dialkylamino, C₃-C₆-Cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₂-alkyl)silyl
oder
R⁹ steht unabhängig voneinander für Phenyl oder Benzyl, das bis zu drei Substituenten enthalten kann, wobei die Substituenten unabhängig voneinander aus folgender Liste ausgewählt sind: C₁-C₃-Alkyl, Halogen, Cyano, Nitro, C₂-C₃-Alkenyl, C₂-C₃-Alkinyl, Cyclopropyl, C₁-C₃-Halogenalkyl, C₂-C₃-Halogenalkenyl, C₂-C₃-Halogenalkinyl, Halogencyclopropyl, C₁-C₂-Alkoxy oder C₁-C₂-Halogenalkoxy
sowie agrochemisch wirksame Salze davon.

3. Verfahren zur Bekämpfung von pflanzenpathogenen Schadpilzen, **dadurch gekennzeichnet, dass** man Verbindungen der Formel (I) gemäß einem der Ansprüche 1 oder 2, auf die pflanzenpathogenen Schadpilze und/oder deren Lebensraum ausbringt.

4. Mittel zur Bekämpfung von pflanzenpathogenen Schadpilzen, **gekennzeichnet durch** einen Gehalt an mindestens einer Verbindung der Formel (I) gemäß einem der Ansprüche 1 oder 2, neben Streckmitteln und/oder oberflächenaktiven Stoffen.

5. Verwendung von Bis(difluormethyl)pyrazol-Derivaten der Formel (I), gemäß einem der Ansprüche 1 oder 2 zur Bekämpfung von pflanzenpathogenen Schadpilzen.

6. Verfahren zur Herstellung von Mitteln zur Bekämpfung von pflanzenpathogenen Schadpilzen, **dadurch gekennzeichnet, dass** man Bis(difluormethyl)pyrazol-Derivate der Formel (I) gemäß einem der Ansprüche 1 oder 2 mit Streckmitteln und/oder oberflächenaktiven Stoffen vermischt.

7. Verwendung von Verbindungen der Formel (I) gemäß einem der Ansprüche 1 oder 2 zur Behandlung von Saatgut.

8. Verwendung von Verbindungen der Formel (I) gemäß einem der Ansprüche 1 oder 2 zur Behandlung von transgenen Pflanzen.

9. Verwendung von Verbindungen der Formel (I) gemäß einem der Ansprüche 1 oder 2 zur Behanlung von transgenem Saatgut.

## Claims

1. Compounds of the formula (I) 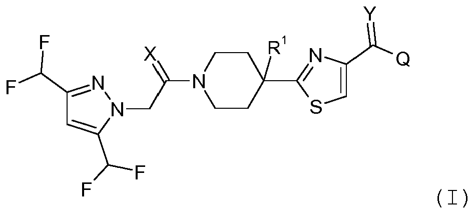 in which the symbols are each defined as follows:
X is oxygen or sulphur,
R¹ is hydrogen or halogen,
Y is oxygen or sulphur,
Q is -NR₂R₃,
R² is hydrogen, C₁-C₃-alkyl, C₂-C₃-alkenyl, C₂-C₃-alkynyl, C₄-C₁₀-cycloalkylalkyl, C₄-C₁₀-alkylcycloalkyl, C₅-C₁₀-alkylcycloalkylalkyl, C₁-C₃-haloalkyl, C₂-C₃-haloalkenyl, cyano, hydroxyl, C₁-C₃-alkoxy, C₂-C₃-alkoxyalkyl, C₁-C₃-hydroxyalkyl, C₂-C₃-alkylcarbonyl, C₂-C₃-alkoxycarbonyl, C₂-C₃-alkylaminocarbonyl or C₃-C₅-dialkylaminocarbonyl,
R³ is an 8- to 11-membered, unsubstituted or substituted, saturated or partly saturated bicyclic ring system or is a 10- to 15-membered, unsubstituted or substituted, saturated or partly saturated tricyclic ring system; each ring system may optionally contain one to three heteroatoms (up to one oxygen atom, up to one sulphur atom and up to three nitrogen atoms) and optionally one to three ring members which may be selected from the group of C(=O), C(=S), S(=O) and S(=O)₂
or
R² and R³ form, together with the nitrogen atom to which they are bonded, a 5- or 6-membered, unsubstituted or substituted, saturated or partly saturated heterocyclyl radical
or
R³ is -CR⁴R⁵R⁶,
R⁴ is hydrogen, C₁-C₆-alkyl, C₂-C₆-alkenyl, C₂-C₆-alkynyl, C₃-C₆-cycloalkyl, C₄-C₁₀-cycloalkylalkyl, C₄-C₁₀-alkylcycloalkyl, C₅-C₁₀-alkylcycloalkylalkyl, C₁-C₆-haloalkyl, C₂-C₆-haloalkenyl, C₂-C₆-haloalkynyl, C₃-C₆-halocycloalkyl, cyano, nitro, C₂-C₄-alkoxyalkyl, C₁-C₄-hydroxyalkyl, C₂-C₄-alkylcarbonyl, C₂-C₆-alkoxycarbonyl, C₂-C₆-alkylaminocarbonyl, C₃-C₈-dialkylaminocarbonyl or C₃-C₆-trialkylsilyl,
R⁵ is unsubstituted or substituted phenyl, benzyl, naphthalenyl, C₃-C₆-cycloalkyl, C₃-C₆-cycloalkenyl or a 5- or 6-membered heteroaryl radical,
R⁶ is hydrogen, C₁-C₄-alkyl, C₂-C₄-alkenyl, C₂-C₄-alkynyl, C₃-C₄-cycloalkyl, C₄-C₁₀-cycloalkylalkyl, C₄-C₁₀-alkylcycloalkyl, C₅-C₁₀-alkylcycloalkylalkyl, C₁-C₄-haloalkyl, C₂-C₄-haloalkenyl, C₂-C₄-haloalkynyl, C₃-C₄-halocycloalkyl or C₂-C₄-alkoxyalkyl,
or
R² and R⁴ form, together with the atoms to which they are bonded, a 5- to 7-membered, unsubstituted or substituted ring system consisting of two to seven carbon atoms and optionally one to three heteroatoms (up to one oxygen atom, up to one sulphur atom and up to two nitrogen atoms),
or
R² and R⁵ form, together with the atoms to which they are bonded, a 5- to 7-membered, unsubstituted or substituted ring system consisting of two to seven carbon atoms and optionally one to three heteroatoms (up to one oxygen atom, up to one sulphur atom and up to two nitrogen atoms),
or
R⁴ and R⁶ form, together with the carbon to which they are bonded, a 5- to 7-membered, unsubstituted or substituted ring system consisting of two to seven carbon atoms and optionally one to three heteroatoms (up to one oxygen atom, up to one sulphur atom and up to one nitrogen atom),
or
R⁴ and R⁵ form, together with the carbon to which they are bonded, a 5- to 7-membered, unsubstituted or substituted ring system consisting of two to seven carbon atoms and optionally one to three heteroatoms (up to one oxygen atom, up to one sulphur atom and up to one nitrogen atom),
and agrochemically active salts thereof.

2. Compounds of the formula (I) according to Claim 1, in which the symbols are each defined as follows:
X is oxygen,
R¹ is hydrogen or fluorine,
Y is oxygen,
Q is 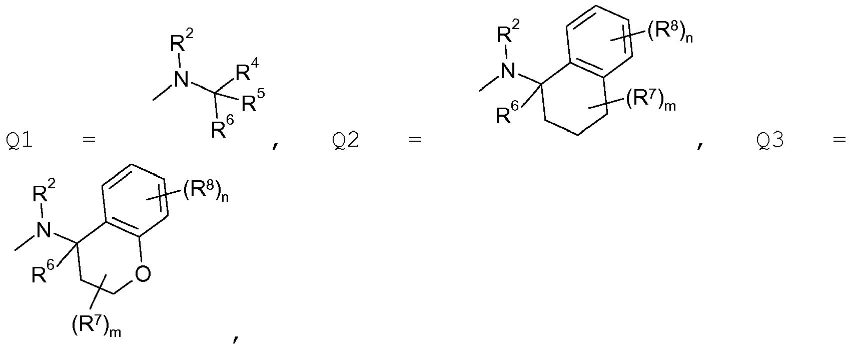 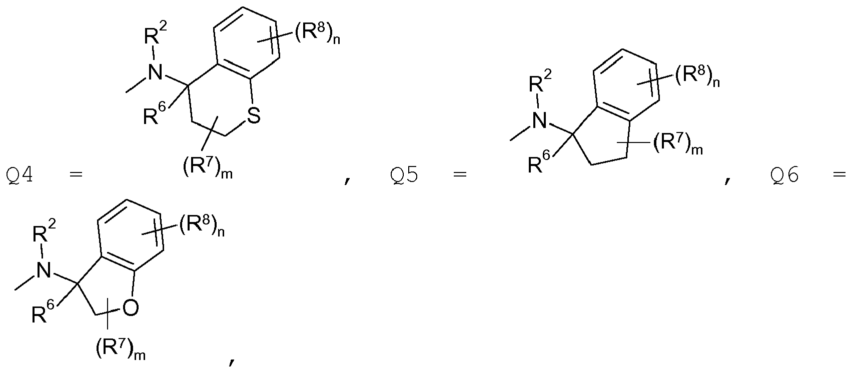 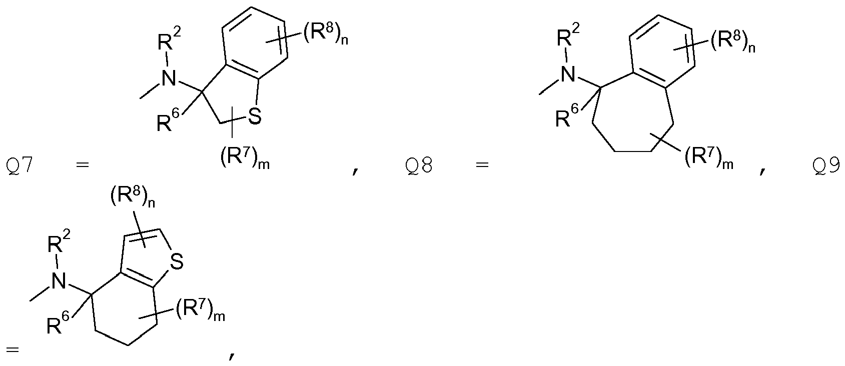 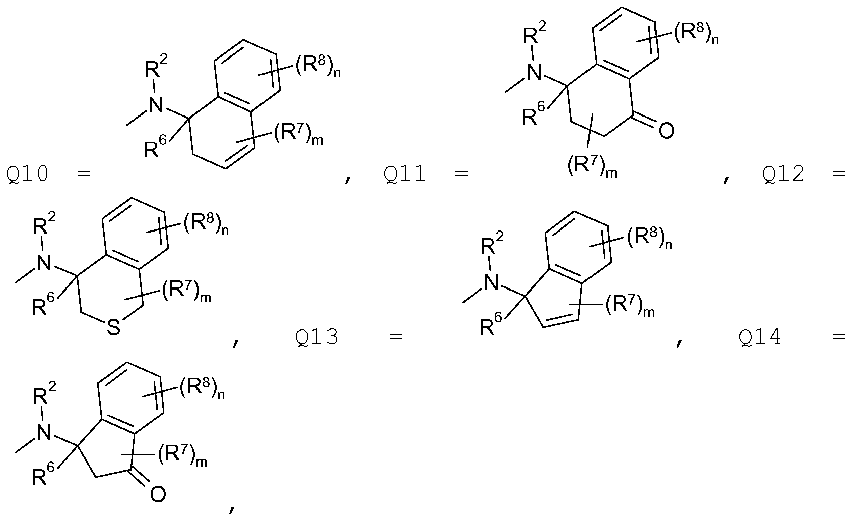 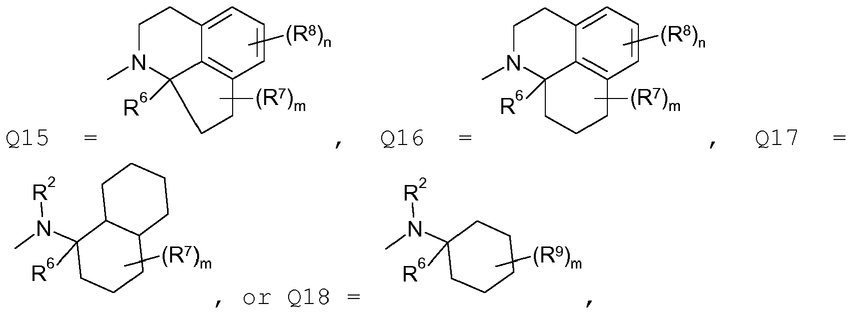
m is 0, 1 or 2,
n is 0, 1 or 2,
R² is hydrogen or C₁-C₃-alkyl,
R⁴ is hydrogen, C₁-C₄-alkyl, C₃-C₆-cycloalkyl, C₂-C₄-alkoxyalkyl, C₁-C₆-haloalkyl,
R⁵ is an unsubstituted or substituted phenyl, benzyl or C₃-C₆-cycloalkyl,
R⁶ is hydrogen or C₁-C₄-alkyl, C₃-C₄-cycloalkyl, C₂-C₄-alkoxyalkyl, C₁-C₄-haloalkyl,
R⁷ is in each case independently hydrogen, C₁-C₄-alkyl, halogen, cyano, hydroxyl, amino, nitro, C₂-C₄-alkenyl, C₃-C₄-alkynyl, C₁-C₄-haloalkyl, C₂-C₄-haloalkenyl, C₂-C₄-haloalkynyl, C₃-C₆-cycloalkyl, C₃-C₆-halocycloalkyl, C₂-C₄-alkoxyalkyl, C₁-C₄-alkylamino, C₂-C₆-dialkylamino, C₃-C₆-cycloalkylamino, C₂-C₄-alkylcarbonyl, C₂-C₄-alkoxycarbonyl, C₂-C₄-alkylaminocarbonyl, C₂-C₄-alkylaminocarbonyloxy, C₃-C₆-dialkylaminocarbonyl, C₁-C₄-hydroxyalkyl, C₁-C₄-alkoxy, C₁-C₄-haloalkoxy, C₂-C₄-alkylcarbonyloxy, C₂-C₄-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl or tri(C₁-C₂-alkyl)silyl,
R⁸ is in each case independently C₁-C₆-alkyl, halogen, cyano, hydroxyl, amino, nitro, C₂-C₆-alkenyl, C₃-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₂-C₄-alkoxyalkyl, C₁-C₄-alkylamino, C₂-C₈-dialkylamino, C₃-C₆-cycloalkylamino, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl or tri(C₁-C₂-alkyl) silyl,
R⁹ is independently C₁-C₆-alkyl, halogen, cyano, hydroxyl, amino, nitro, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₄-C₁₀-cycloalkylalkyl, C₄-C₁₀-alkylcycloalkyl, C₅-C₁₀-alkylcycloalkylalkyl, C₃-C₆-halocycloalkyl, C₂-C₄-alkoxyalkyl, C₁-C₄-alkylamino, C₂-C₈-dialkylamino, C₃-C₆-cycloalkylamino, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₂-alkyl)silyl
or
R⁹ is independently phenyl or benzyl which may contain up to three substituents, where the substituents are each independently selected from the following list: C₁-C₃-alkyl, halogen, cyano, nitro, C₂-C₃-alkenyl, C₂-C₃-alkynyl, cyclopropyl, C₁-C₃-haloalkyl, C₂-C₃-haloalkenyl, C₂-C₃-haloalkynyl, halocyclopropyl, C₁-C₂-alkoxy or C₁-C₂-haloalkoxy
and agrochemically active salts thereof.

3. Method for controlling phytopathogenic harmful fungi, **characterized in that** compounds of the formula (I) according to either of Claims 1 and 2 are applied to the phytopathogenic harmful fungi and/or their habitat.

4. Composition for controlling phytopathogenic harmful fungi, **characterized by** a content of at least one compound of the formula (**I**) according to either of Claims 1 and 2, in addition to extenders and/or surfactants.

5. Use of bis(difluoromethyl)pyrazole derivatives of the formula (**I**) according to either of Claims 1 and 2 for controlling phytopathogenic harmful fungi.

6. Process for producing compositions for controlling phytopathogenic harmful fungi, **characterized in that** bis(difluoromethyl)pyrazole derivatives of the formula (**I**) according to either of Claims 1 and 2 are mixed with extenders and/or surfactants.

7. Use of compounds of the formula (I) according to either of Claims 1 and 2 for treatment of seed.

8. Use of compounds of the formula (I) according to either of Claims 1 and 2 for treatment of transgenic plants.

9. Use of compounds of the formula (I) according to either of Claims 1 and 2 for treatment of transgenic seed.

## Revendications

1. Composés de formule (I) 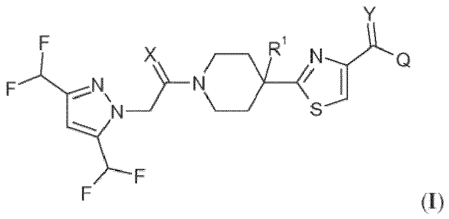 dans lesquels les symboles ont les significations suivantes :
X représente oxygène ou soufre,
R¹ représente hydrogène ou halogène,
Y représente oxygène ou soufre,
Q représente -NR²R³,
R² représente hydrogène, alkyle en C₁-C₃, alcényle en C₂-C₃, alcynyle en C₂-C₃, cycloalkylalkyle en C₄-C₁₀, alkylcycloalkyle en C₄-C₁₀, alkylcycloalkylalkyle en C₅-C₁₀, halogénoalkyle en C₁-C₃, halogénoalcényle en C₂-C₃, cyano, hydroxy, alcoxy en C₁-C₃, alcoxyalkyle en C₂-C₃, hydroxyalkyle en C₁-C₃, alkylcarbonyle en C₂-C₃, alcoxycarbonyle en C₂-C₃, alkylaminocarbonyle en C₂-C₃ ou dialkylaminocarbonyle en C₃-C₅,
R³ représente un système cyclique bicyclique de 8 à 11 éléments, non substitué ou substitué, saturé ou partiellement saturé, ou représente un système cyclique tricyclique de 10 à 15 éléments, non substitué ou substitué, saturé ou partiellement saturé ; chaque système cyclique pouvant éventuellement contenir un à trois hétéroatomes (jusqu'à un atome d'oxygène, jusqu'à un atome de soufre et jusqu'à trois atomes d'azote) et éventuellement un à trois éléments de cycle, qui peuvent être choisis dans le groupe constitué par C(=O), C(=S), S(=O) ou S(=O)₂,
ou
R² et R³ forment ensemble avec l'atome d'azote auquel ils sont reliés un radical hétérocyclyle à 5 ou 6 éléments, non substitué ou substitué, saturé ou partiellement saturé,
ou
R³ représente -CR⁴R⁵R⁶,
R⁴ représente hydrogène, alkyle en C₁-C₆, alcényle en C₂-C₆, alcynyle en C₂-C₆, cycloalkyle en C₃-C₆, cycloalkylalkyle en C₄-C₁₀, alkylcycloalkyle en C₄-C₁₀, alkylcycloalkylalkyle en C₅-C₁₀, halogénoalkyle en C₁-C₆, halogénoalcényle en C₂-C₆, halogénoalcynyle en C₂-C₆, halogénocycloalkyle en C₃-C₆, cyano, nitro, alcoxyalkyle en C₂-C₄, hydroxyalkyle en C₁-C₄, alkylcarbonyle en C₂-C₄, alcoxycarbonyle en C₂-C₆, alkylaminocarbonyle en C₂-C₆, dialkylaminocarbonyle en C₃-C₈ ou trialkylsilyle en C₃-C₆,
R⁵ représente phényle, benzyle, naphtalényle, cycloalkyle en C₃-C₆, cycloalcényle en C₃-C₆ ou un radical hétéroaryle à 5 ou 6 éléments, non substitué ou substitué,
R⁶ représente hydrogène, alkyle en C₁-C₄, alcényle en C₂-C₄, alcynyle en C₂-C₄, cycloalkyle en C₃-C₄, cycloalkylalkyle en C₄-C₁₀, alkylcycloalkyle en C₄-C₁₀, alkylcycloalkylalkyle en C₅-C₁₀, halogénoalkyle en C₁-C₄, halogénoalcényle en C₂-C₄, halogénoalcynyle en C₂-C₄, halogénocycloalkyle en C₃-C₄ ou alcoxyalkyle en C₂-C₄,
ou
R² et R⁴ forment ensemble avec les atomes auxquels ils sont reliés un système cyclique de 5 à 7 éléments, non substitué ou substitué, constitué de deux à sept atomes de carbone et éventuellement un à trois hétéroatomes (jusqu'à un atome d'oxygène, jusqu'à un atome de soufre et jusqu'à deux atomes d'azote),
ou
R² et R⁵ forment ensemble avec les atomes auxquels ils sont reliés un système cyclique de 5 à 7 éléments, non substitué ou substitué, constitué de deux à sept atomes de carbone et éventuellement un à trois hétéroatomes (jusqu'à un atome d'oxygène, jusqu'à un atome de soufre et jusqu'à deux atomes d'azote),
ou
R⁴ et R⁶ forment ensemble avec le carbone auquel ils sont reliés un système cyclique de 5 à 7 éléments, non substitué ou substitué, constitué de deux à sept atomes de carbone et éventuellement un à trois hétéroatomes (jusqu'à un atome d'oxygène, jusqu'à un atome de soufre et jusqu'à un atome d'azote),
ou
R⁴ et R⁵ forment ensemble avec le carbone auquel ils sont reliés un système cyclique de 5 à 7 éléments, non substitué ou substitué, constitué de deux à sept atomes de carbone et éventuellement un à trois hétéroatomes (jusqu'à un atome d'oxygène, jusqu'à un atome de soufre et jusqu'à un atome d'azote),
ainsi que leurs sels agrochimiquement efficaces.

2. Composés de formule (I) selon la revendication 1, dans lesquels les symboles ont les significations suivantes :
X représente oxygène,
R¹ représente hydrogène ou fluor,
Y représente oxygène,
Q représente 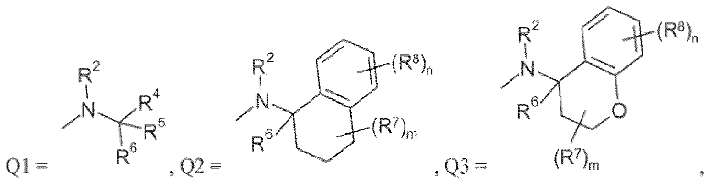 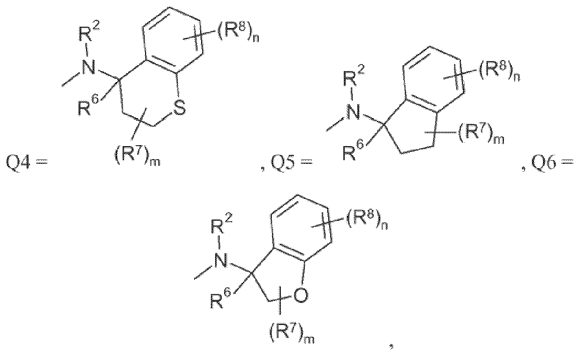 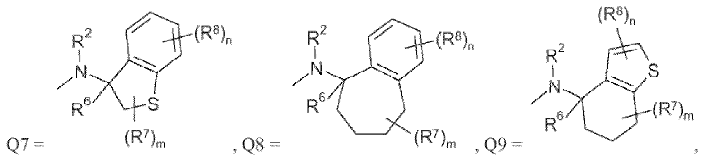 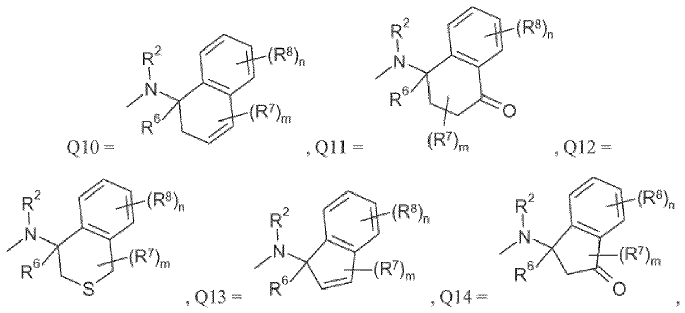 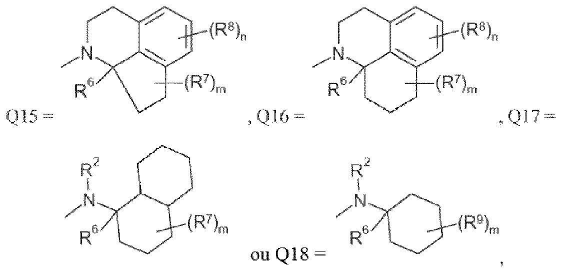
m représente 0, 1 ou 2,
n représente 0, 1 ou 2,
R² représente hydrogène ou alkyle en C₁-C₃,
R⁴ représente hydrogène, alkyle en C₁-C₄, cycloalkyle en C₃-C₆, alcoxyalkyle en C₂-C₄, halogénoalkyle en C₁-C₆,
R⁵ représente phényle, benzyle ou cycloalkyle en C₃-C₆, non substitué ou substitué,
R⁶ représente hydrogène ou alkyle en C₁-C₄, cycloalkyle en C₃-C₄, alcoxyalkyle en C₂-C₄, halogénoalkyle en C₁-C₄,
les R⁷ représentent chacun indépendamment les uns des autres hydrogène, alkyle en C₁-C₄, halogène, cyano, hydroxy, amino, nitro, alcényle en C₂-C₄, alcynyle en C₃-C₄, halogénoalkyle en C₁-C₄, halogénoalcényle en C₂-C₄, halogénoalcynyle en C₂-C₄, cycloalkyle en C₃-C₆, halogénocycloalkyle en C₃-C₆, alcoxyalkyle en C₂-C₄, alkylamino en C₁-C₄, dialkylamino en C₂-C₆, cycloalkylamino en C₃-C₆, alkylcarbonyle en C₂-C₄, alcoxycarbonyle en C₂-C₄, alkylaminocarbonyle en C₂-C₄, alkylaminocarbonyloxy en C₂-C₄, dialkylaminocarbonyle en C₃-C₆, hydroxyalkyle en C₁-C₄, alcoxy en C₁-C₄, halogénoalcoxy en C₁-C₄, alkylcarbonyloxy en C₂-C₄, alkylcarbonylthio en C₂-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄ ou tri (alkyle en C₁-C₂) silyle,
les R⁸ représentent chacun indépendamment les uns des autres alkyle en C₁-C₆, halogène, cyano, hydroxy, amino, nitro, alcényle en C₂-C₆, alcynyle en C₃-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alcoxyalkyle en C₂-C₄, alkylamino en C₁-C₄, dialkylamino en C₂-C₈, cycloalkylamino en C₃-C₆, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄ ou tri (alkyle en C₁-C₂) silyle,
les R⁹ représentent indépendamment les uns des autres alkyle en C₁-C₆, halogène, cyano, hydroxy, amino, nitro, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, cycloalkylalkyle en C₄-C₁₀, alkylcycloalkyle en C₄-C₁₀, alkylcycloalkylalkyle en C₅-C₁₀, halogénocycloalkyle en C₃-C₆, alcoxyalkyle en C₂-C₄, alkylamino en C₁-C₄, dialkylamino en C₂-C₈, cycloalkylamino en C₃-C₆, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, tri (alkyle en C₁-C₂) silyle,
ou
les R⁹ représentent indépendamment les uns des autres phényle ou benzyle, qui peut contenir jusqu'à trois substituants, les substituants étant choisis indépendamment les uns des autres dans la liste suivante : alkyle en C₁-C₃, halogène, cyano, nitro, alcényle en C₂-C₃, alcynyle en C₂-C₃, cyclopropyle, halogénoalkyle en C₁-C₃, halogénoalcényle en C₂-C₃, halogénoalcynyle en C₂-C₃, halogénocyclopropyle, alcoxy en C₁-C₂ ou halogénoalcoxy en C₁-C₂, ainsi que leurs sels agrochimiquement efficaces.

3. Procédé de lutte contre les champignons phytopathogènes, **caractérisé en ce que** des composés de formule (I) selon l'une quelconque des revendications 1 ou 2 sont appliqués sur les champignons phytopathogènes et/ou leur habitat.

4. Agent de lutte contre les champignons phytopathogènes, **caractérisé par** une teneur en au moins un composé de formule (I) selon l'une quelconque des revendications 1 ou 2, en plus d'extendeurs et/ou de substances tensioactives.

5. Utilisation de dérivés de bis(difluorométhyl)pyrazole de formule (I) selon l'une quelconque des revendications 1 ou 2 pour lutter contre les champignons phytopathogènes.

6. Procédé de fabrication d'agents de lutte contre les champignons phytopathogènes, **caractérisé en ce que** des dérivés de bis(difluorométhyl)pyrazole de formule (I) selon l'une quelconque des revendications 1 ou 2 sont mélangés avec des extendeurs et/ou des substances tensioactives.

7. Utilisation de composés de formule (I) selon l'une quelconque des revendications 1 ou 2 pour le traitement de graines.

8. Utilisation de composés de formule (I) selon l'une quelconque des revendications 1 ou 2 pour le traitement de plantes transgéniques.

9. Utilisation de composés de formule (I) selon l'une quelconque des revendications 1 ou 2 pour le traitement de graines transgéniques.
